# EUROPEAN PATENT SPECIFICATION

(11) **EP 3 998 338 B1**
(45) Date of publication and mention of the grant of the patent: **24.04.2024**
(21) Application number: 20836410.9
(22) Date of filing: 10.07.2020
(51) Int. Cl.: C12Q 1/6869, C12Q 1/6806

(54) **METHOD FOR AMPLIFYING NUCLEIC ACID USING SOLID-PHASE CARRIER**
VERFAHREN ZUR AMPLIFIZIERUNG VON NUKLEINSÄUREN UNTER VERWENDUNG EINES FESTPHASENTRÄGERS
PROCÉDÉS D'AMPLIFICATION D'ACIDE NUCLÉIQUE À L'AIDE D'UN SUPPORT EN PHASE SOLIDE

(30) Priority: 11.07.2019 JP 2019129017
(43) Date of publication of application: 18.05.2022
(73) Proprietor: Tokyo University of Science Foundation, Tokyo 162-8601 (JP)
(72) Inventor: MATSUSHIMA, Kouji, Tokyo 162-8601 (JP); UEHA, Satoshi, Tokyo 162-8601 (JP); SHICHINO, Shigeyuki, Tokyo 162-8601 (JP); ITO, Satoru, Tokyo 162-8601 (JP); AOKI, Hiroyasu, Tokyo 162-8601 (JP)
(74) Representative: Mewburn Ellis LLP
(86) International application number: PCT/JP2020/027123
(87) International publication number: WO 2021/006353

(56) References cited:
- WO-A1-2013/145431
- WO-A1-2014/031954
- WO-A1-2015/166768
- WO-A1-2016/172373
- WO-A1-2018/089550
- WO-A2-2014/108850
- JP-A- 2016 533 187
- JP-A- 2017 522 867
- US-A1- 2015 024 959
- US-A1- 2015 376 609
- US-A1- 2016 312 276
- US-A1- 2018 251 825
- SIMONE PICELLI: "Single-cell RNA-sequencing: The future of genome biology is now", RNA BIOLOGY, vol. 14, no. 5, 21 July 2016 (2016-07-21), pages 637-650, XP055454999, ISSN: 1547-6286, DOI: 10.1080/15476286.2016.1201618
- Vanessa M Peterson, Kelvin Xi Zhang, Namit Kumar, Jerelyn Wong, Lixia Li, Douglas C Wilson, Renee Moore, Terrill K Mcclanahan, Sve: "Multiplexed quantification of proteins and transcripts in single cells", Nature Biotechnology, Gale Group Inc., New York, us, vol. 35, no. 10 , pages 936-939, XP055587549, us ISSN: 1087-0156, DOI: 10.1038/nbt.3973
- Marlon Stoeckius, Hafemeister Christoph, Stephenson William, Houck-Loomis Brian, Chattopadhyay Pratip K, Swerdlow Harold, Satija R: "Simultaneous epitope and transcriptome measurement in single cells", Nature Methods, Nature Pub. Group, New York, vol. 14, no. 9, 1 September 2017 (2017-09-01), pages 865-868, XP055547724, New York ISSN: 1548-7091, DOI: 10.1038/nmeth.4380
- Aoki Hiroyasu, Ueha Satoshi, Shichino Shigeyuki, Ogiwara Haru, Hashimoto Shin-Ichi, Kakimi Kazuhiro, Ito Satoru, Matsushima Kouji: "TCR Repertoire Analysis Reveals Mobilization of Novel CD8+ T Cell Clones Into the Cancer-Immunity Cycle Following Anti-CD4 Antibody Administration", Frontiers in Immunology, vol. 9, 1 January 2018 (2018-01-01), page 3185, XP055785297, DOI: 10.3389/fimmu.2018.03185

## Description

### TECHNICAL FIELD

The present invention relates to a method for nucleic acid amplification using a solid-phase carrier.

### BACKGROUND ART

Recently, comprehensive gene expression analysis has been carried out using small amounts of samples such as single individual cells. Such a technique has enabled single-cell RNA-seq analysis (single cell transcriptome (SCT) analysis) in which properties of several hundred to several ten thousand individual cells are comprehensively understood, thereby enabling identification of the presence or absence of diversity within a cell population, identification of novel cell subpopulations, and identification of special cell populations related to pathological conditions. Furthermore, for example, an international project aiming at preparation of a single-cell-level atlas in humans has started in 2016 (Non-Patent Document 1). The technique is thus serving as a driving force for understanding of biology with a fine resolution that has never achieved before.

Other representative examples of the small amounts of samples include tumor-infiltrating T cells contained in biopsy specimens of primary lesions and metastatic lesions collected for the purpose of diagnosis in cancer patients. From the viewpoint of the fact that CTLs are essential in immunotherapies using anti-PD-1 antibody or the like, and that they are actually CD8⁺ T cells, methods of measuring the extent to which CD8⁺ T cells capable of specific recognition of cancer antigens are induced are attracting attention (Non-Patent Document 2). In one of such measurement methods, the sequences of T cell receptors (TCRs) on CD8⁺ T cells are analyzed to determine the TCR repertoire (the clone types and the abundances of the clones) to discuss the responses of tumor-specific clones. It has been reported that the therapeutic effect of anti-CD4 antibody on cancer is actually correlated with change in the TCR repertoire (Non-Patent Document 3). However, most of the cells of tumor-infiltrating T cells are cancer cells and tissue cells, and the content of T cells is very low in most cases. Therefore, for SCT analysis, and for robust TCR repertoire analysis of cancer sites, development of a highly sensitive, high-throughput method for amplifying mRNA extracted from a specimen or for amplifying mRNA derived from individual T cells derived from a specimen is now becoming more important.

Examples of methods of amplifying mRNA from a small amount of sample include liquid-phase systems in which the amplification is carried out after dispensing individual cells or a small amount of mRNA in a 96-well plate, such as Smart-Seq2 (Patent Document 1 and Non-Patent Document 4); droplet systems in which droplets are formed using a microchannel, and a single cell is captured in each droplet, such as Chromium by 10X Genomics (Patent Document 2); and methods in which mRNA in each sample is captured using a solid-phase bead. Although Smart-Seq2 is highly sensitive, sorting of individual cells is carried out in a 96-well or 384-well plate using a flow cytometer, so that the throughput is low, and the cost is high. On the other hand, droplet systems such as 10X Chromium are high-throughput systems, but they show much lower sensitivities compared to Smart-Seq2, which is problematic (Non-Patent Document 5). Moreover, in both liquid-phase systems and droplet systems, carry-over of the reaction liquid occurs, so that surfactants having strong protein-denaturing action cannot be used as a reagent for cell lysis. Therefore, analysis of, for example, cells comprising a large amount of ribonuclease is difficult, which is problematic.

mRNA/cDNA amplification methods through immobilization on beads enable simple replacement of the reaction liquid between reaction steps, without causing extensive loss of the substrate cDNA. Therefore, the methods are compatible with a wider range of reaction conditions compared to liquid-phase systems, and hence enable, for example, use of a surfactant having strong protein-denaturing action. Further, by using a magnetic bead as a solid-phase bead carrier, automation or semi-automation of a process using a magnetic stand and an automatic pipetting workstation is possible, so that the methods provide a useful technique for high-throughput SCT/TCR analysis. In practice, Kambara et al. showed that a small amount of, or single-cell-derived, cDNA can be amplified by using a magnetic bead (Patent Document 3 and Non-Patent Document 6). However, the above past techniques require strict control of parameters for poly(A) addition reaction utilizing terminal deoxynucleotidyl transferase (TdT) (the ratio between the amount of DNA substrate on the magnetic bead and the amount of enzyme, the reaction time, the temperature, and the like). For example, in cases where the primer density on the magnetic bead is too high, a large amount of primer-derived reaction by-product is produced by TdT, leading to a significant decrease in the cDNA amplification efficiency (Non-Patent Document 7). Further, in cases where the reaction time with TdT is too long, excessive elongation of the primer-derived by-product occurs, so that the by-product cannot be isolated from the cDNA (Non-Patent Document 8). Thus, the techniques have a problem in that they are largely affected by the lot-to-lot variation of the TdT enzyme activity, differences in the primer content and the cDNA content in the reaction system, the operational time lag in the automatic pipetting system, and the like. Moreover, although comprehensive single-cell-derived mRNA analysis techniques using beads widely employ exonuclease I for elimination of unreacted primers comprising cell barcodes, the exonuclease I treatment cannot be carried out in the highly sensitive amplification method by Kambara et al. since the treatment changes the ratio between the number of DNA substrate molecules and the amount of TdT enzyme, which is problematic.

In a recently developed method that is commercially available from Biolegend (https://www.biolegend.com/en-us/totalseq) and the like, antibodies that specifically recognize the respective proteins are labeled with oligo DNAs, to enable simultaneous quantification of the expression levels of several ten proteins expressed on each single cell together with the transcriptome of each single cell. In this method, the expression level of each protein is analyzed as the expression level of a DNA tag for each antibody according to sequence analysis. Further, by targeting a protein widely expressed in various types of cells, and employing different kinds of DNA tags, the method is also applicable to separation of single-cell transcriptome data of each sample from a mixture of a plurality of samples. The method therefore enables cost reduction, batch effect reduction, analysis of biological replicates, and the like (https://www.biolegend.com/en-us/bio-bits/new-cell-hashtag-reagents-for-single-cell-analysis). Such single-cell transcriptome analysis in combination with cell labeling with a DNA-labeled antibody may be applicable to a variety of uses. However, although the DNA tag is an oligo DNA, it has a length of not less than 84 base pairs, which is relatively long, and its combination with a cell barcode used for the single-cell transcriptome results in a length of as long as not less than 145 base pairs. On the other hand, it has been reported that, in the conventional TdT method, in cases where relatively long DNA fragments such as a primer of not less than 126 base pairs are present, cDNAs cannot be efficiently separated from those products even though the lengths of the DNA fragments are shorter than the cDNAs (Non-Patent Document 9). Since the expression levels of proteins are generally much higher than the expression levels of mRNAs, separation of DNA tag products for detection of proteins from amplified cDNAs has been difficult in the conventional TdT method.

An SCT analysis system using a solid-phase magnetic bead and a microwell is commercially available from Beckton Dickinson under the name BD Rhapsody System (Patent Document 4). However, since the RNA analysis method by Beckton Dickinson only provides a poorly sensitive amplification system whose efficiency is almost equivalent to those of gene analysis panels carrying only about 500 genes or Chromium manufactured by 10X Genomics. Thus, the system still does not meet the needs of researchers. Other examples of SCT analysis methods using a solid-phase bead include the Drop-seq method (Non-Patent Document 10) and the Seq-well method (Non-Patent Document 11). Since these methods use the SMART method, which is less efficient than the TdT method, their low sensitivities are problematic. Patent Document 5 relates to methods for whole transcriptome amplification using stochastic barcodes conjugated to beads. Patent Document 6 relates to methods for synthesizing cDNA comprising combining an RNA sample with a polydT oligonucleotide attached to a solid-phase, and incubating with reverse transcriptase comprising Terminal Deoxynucleotidyl Transferase (TdT). Patent Document 7 relates to methods of amplifying nucleic acid molecules mediated by the addition of a homopolymer.

### PRIOR ART DOCUMENTS

### PATENT DOCUMENTS

Patent Document 1: WO 2015/027135 A1
Patent Document 2: WO 2015/200893 A2
Patent Document 3: WO 2013/145431 A1
Patent Document 4: US 2018/0258500 A1
Patent Document 5: WO 2016/172373 A1
Patent Document 6: WO 2014/108850 A2
Patent Document 7: WO 2014/031954 A1

### NON-PATENT DOCUMENTS

Non-Patent Document 1: Regev A., et al. The Human Cell Atlas. eLife 2017;e27041.
Non-Patent Document 2: Zaretsky JM, Garcia-Diaz A, Shin DS, et al. Mutations Associated with Acquired Resistance to PD-1 Blockade in Melanoma. N Engl J Med 2016;375:819-2.
Non-Patent Document 3: Aoki H., et al. TCR Repertoire Analysis Reveals Mobilization of Novel CD8+ T Cell Clones Into the Cancer-Immunity Cycle Following Anti-CD4 Antibody Administration. Front Immunol. 2019 Jan; 9(3185). Non-Patent Document 4: Picelli S., et al. Full-length RNA-seq from single cells using Smart-seq2. Nat Protoc. 2014 Jan;9(1):171-81.
Non-Patent Document 5: Single-cell transcriptomics of 20 mouse organs creates a Tabula Muris. Nature. 2018 Oct;562(7727):367-372.
Non-Patent Document 6: Matsunaga H, Goto M, Arikawa K, et al. A highly sensitive and accurate gene expression analysis by sequencing ("bead-seq") for a single cell. Anal Biochem 2015;471:9-16.
Non-Patent Document 7: Huan Huang, Mari Goto, Hiroyuki Tsunoda, Lizhou Sun, Kiyomi Taniguchi, Hiroko Matsunaga, Hideki Kambara. Non-biased and efficient global amplification of a single-cell cDNA library. Nucleic Acid Research 2013.
Non-Patent Document 8: Sasagawa Y, Nikaido I, Hayashi T, Danno H, Uno KD, Imai T, Ueda HR. Quartz-Seq: a highly reproducible and sensitive single-cell RNA sequencing method, reveals non-genetic gene-expression heterogeneity. Genome Biol 2013.
Non-Patent Document 9: Yohei Sasagawa, Hiroki Danno, Hitomi Takada, Masashi Ebisawa, Kaori Tanaka, Tetsutaro Hayashi, Akira Kurisaki, and Itoshi Nikaido. Quartz-Seq2: a high-throughput single-cell RNA-sequencing method that effectively uses limited sequence reads. Genome Biol 2018.
Non-Patent Document 10: Macosko EZ et al. Highly Parallel Genome-wide Expression Profiling of Individual Cells Using Nanoliter Droplets. Cell 2015; 161(5): 1202-1214.
Non-Patent Document 11: Gierahn TM et al. Seq-Well: portable, low-cost RNA sequencing of single cells at high throughput. Nat Methods 2017;14(4):395-398.

### SUMMARY OF THE INVENTION

### PROBLEMS TO BE SOLVED BY THE INVENTION

An object of the present invention is to provide a method of amplifying nucleic acid such as cDNA using a solid-phase carrier, which method enables highly efficient, comprehensive amplification of mRNA even from a very small amount of cell sample.

### MEANS FOR SOLVING THE PROBLEMS

A method applicable at clinical sites is to infer the TCR repertoire response of CD8⁺ TILs from the TCR repertoire of CD8⁺ T cells in peripheral blood. Unfortunately, it was found, as a result of study by the present inventors, that information reflecting tumor immune response cannot be obtained by analysis of peripheral blood CD8⁺ T cells alone. Accordingly, the only means that may be applicable at clinical sites is analysis of the change in the repertoire of CD8⁺ TILs without separating the TILs. In other words, if sufficient information on the tumor immune response can be obtained by directly analyzing a tissue biopsy specimen itself, that is, by subjecting a tissue biopsy specimen to analysis without separating TILs, valuable information can be obtained as a result even from a small amount of tissue. Such information is important for determination of the course of treatment in the clinical side. In the BD Rhapsody System, which is a representative SCT analysis platform using a solid-phase carrier, the actual cost required for each time of analysis is several hundred thousand yen, which is extremely expensive. In cases where such an important sample is to be analyzed, it is important to maintain both sensitivity and robustness at high levels for enabling wide clinical application. Unfortunately, it was found, as a result of study by the present inventors, that the existing amplification methods using a bead carrier are sensitive to lot-to-lot variation of reagents, differences in the skill of the experimenter, and the like.

As a result of intensive study, the present inventors discovered that, by using chain-terminating reaction with a chain-terminating nucleotide triphosphate in reaction of nucleotide homopolymer addition to cDNA synthesized on a solid-phase carrier, a small amount of cDNA can be stably and highly efficiently amplified even in cases where the ratio between the amount of DNA substrate and the amount of enzyme on the solid phase is excessive, where the reaction time is excessive, and/or where reagents show lot-to-lot variation. The present inventors also discovered that, by combination of the present method with exonuclease I treatment, remarkable reduction of by-products and an increase in the cDNA amplification efficiency can be achieved. The present inventors further discovered that, even in cases where a DNA product that is shorter than cDNAs but has a relatively long size, derived from the oligo DNA label of an oligo DNA-labeled antibody or the like, is contained, by-products derived from unreacted primers, amplification products derived from the oligo DNA-labeled antibody, and cDNA amplification products can be clearly separated from each other based on the difference in the chain length.

More specifically, the present invention is a method for nucleic acid amplification using a solid-phase carrier as set out in the appended claims.

### EFFECT OF THE INVENTION

In the amplification method of the present invention, chain-terminating reaction with a chain-terminating nucleotide triphosphate which is a chain-terminating CTP or a chain-terminating GTP, such as ddNTP (N is C or G), is used in reaction of nucleotide homopolymer addition to cDNA synthesized on a solid-phase carrier. By this, cDNA can be stably and highly efficiently amplified even from a small amount of sample even in cases where the ratio of the amount of enzyme to the amount of DNA substrate on the solid phase is excessive, where the reaction time is excessive, and/or where reagents show lot-to-lot variation.

Conventional techniques are affected by a difference in the skill level of the experimenter (which tends to cause time lags in the experimental operation, leading to an excessive reaction time in cases where the skill level is low), lot-to-lot variation of reagents, and the like. It is therefore difficult for those techniques to maintain both sensitivity and robustness at high levels. In contrast, according to the method of the present invention, robust, highly sensitive, and comprehensive amplification of cDNA from mRNA is possible even from a small amount of sample that cannot be easily handled, such as single cells or a tumor biopsy specimen, without being affected by the skill level of the experimenter, lot-to-lot variation of reagents, or the like.

Since the method enables reduction of the cost required for learning by the experimenter or for automation, hurdles to widespread use can be lowered.

The method by Kambara et al. (Patent Document 3 and Non-Patent Document 6) is a method capable of highly sensitively amplifying cDNA using magnetic beads. However, the method requires strict control of, for example, parameters for homopolymer addition reaction using TdT (the ratio between the amount of DNA substrate on the magnetic bead and the amount of enzyme, the reaction time, the temperature, and the like). Since exonuclease I treatment changes the ratio between the amount of DNA substrate on the solid phase and the amount of TdT enzyme, unreacted primers cannot be eliminated by exonuclease I in the amplification method by Kambara et al., which is problematic. In contrast, in the method of the present invention, chain-terminating reaction with a chain-terminating CTP or GTP is employed, so that strict control of the parameters for the homopolymer addition reaction by TdT is not necessary, and therefore the unreacted primers can be removed by exonuclease I.

The amplification method of the present invention enables expression analysis and TCR analysis even in cases where the amount of the specimen is small. Therefore, the method can, for example, largely improve the accuracy of judgment of a therapeutic effect utilizing expression analysis and/or TCR analysis, and hence can contribute to suppression of wasteful use of medical resources and the like.

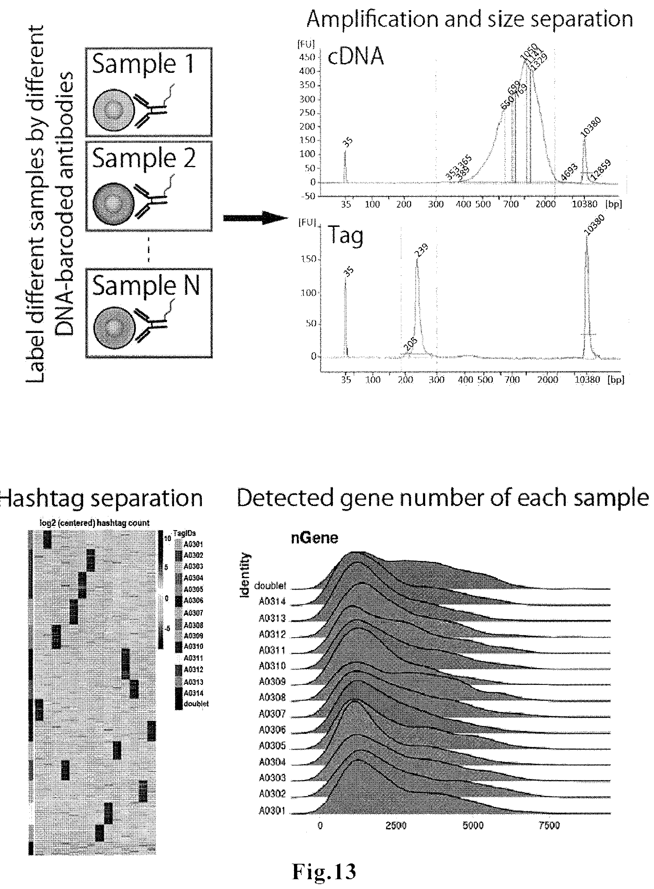

Techniques in which antibodies labeled with oligo DNA tags are used to simultaneously carry out proteome analysis of each single cell and transcriptome analysis of each single cell have been developed, and such techniques are potentially applicable to various uses. However, since the separation of amplification products derived from oligo DNA tags from cDNA amplification products is difficult in the conventional TdT method, applicability of the analysis technique combined with the use of DNA-labeled antibodies has been limited. In contrast, since the amplification method of the present invention enables clear separation between DNA amplification products derived from oligo DNA tags and cDNA amplification products derived from mRNA (see Examples and Fig. 13), the present invention can broaden the range of applications of techniques in which analysis using a specific-binding molecule labeled with an oligonucleic acid, such as a DNA-labeled antibody and analysis of transcripts are carried out simultaneously.

In the amplification method of the present invention, by combined use with oligonucleic acid-labeled antibodies against a plurality of proteins, expression analysis of proteins and mRNAs in each single cell can be simultaneously and highly sensitively carried out. Further, by combined use with an oligonucleic acid-labeled antibody against a cell surface molecule stably expressed on the cells to be analyzed, or with an oligonucleic acid-labeled specific-binding molecule capable of non-specifically labeling cell surface molecules in general and its partner molecule, simultaneous analysis (multiplex analysis) of a plurality of samples is possible, so that the cost required for preparation of cDNA libraries can be reduced proportionally to the number of samples analyzed simultaneously.

### BRIEF DESCRIPTION OF THE DRAWINGS

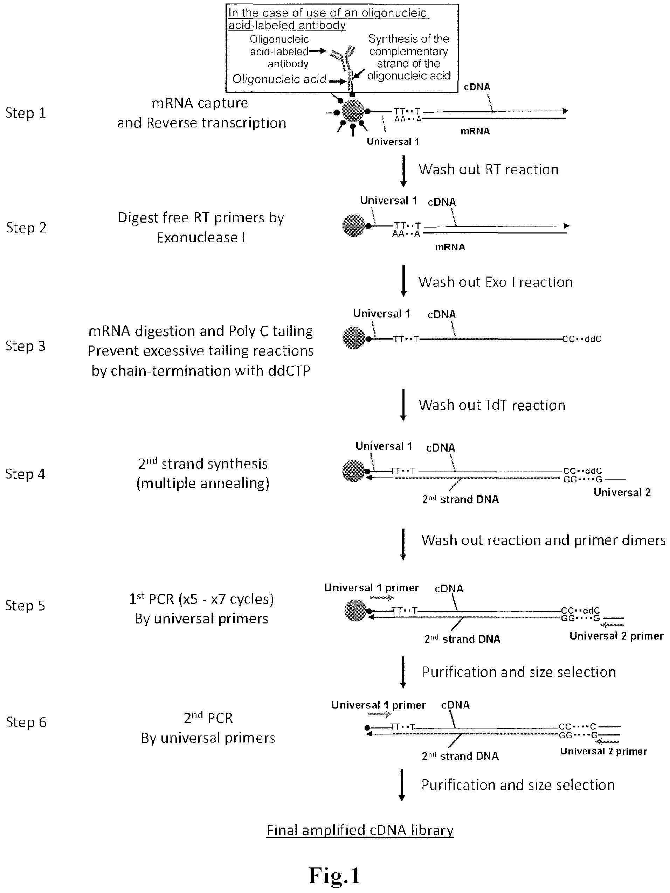

Fig. 1 is a scheme illustrating a method of total cDNA amplification from a bead solid-phase carrier (one example of application to SCT analysis).

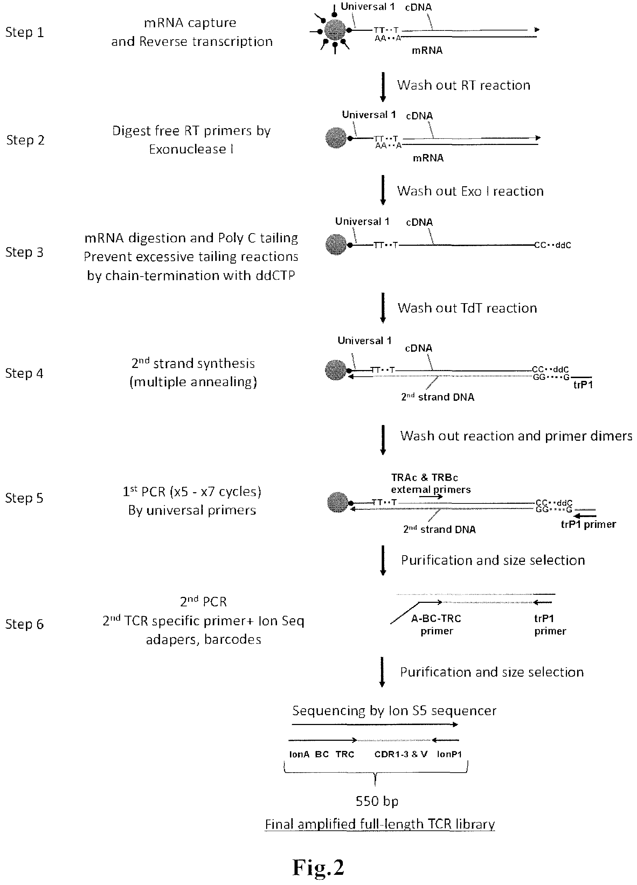

Fig. 2 is a scheme illustrating a method of amplifying TCR cDNA from a bead solid-phase carrier (one example of application to TCR repertoire analysis).

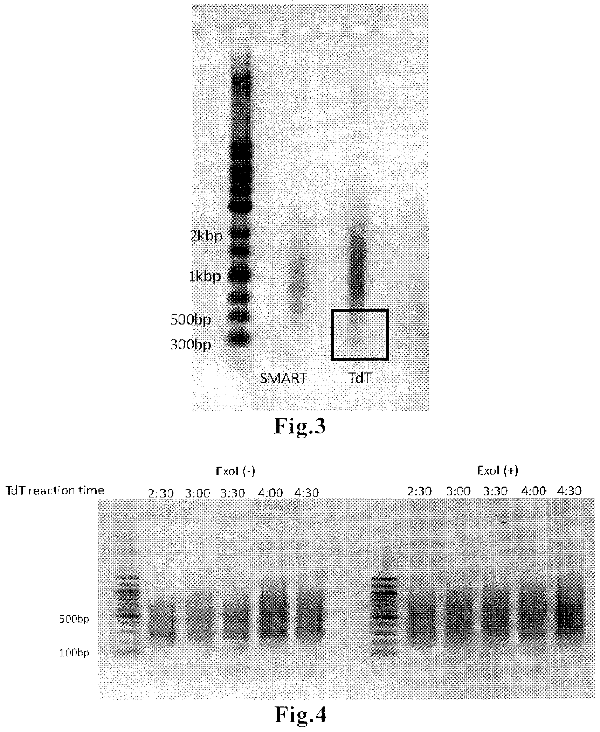

Fig. 3 (not according to the present invention) shows the result of agarose electrophoresis of the products of amplification of total cDNA by the TdT method or the SMART method without using ddNTP and exonuclease I in Nx1-seq. In the TdT method, the yield of amplified cDNA was higher than in the SMART method, but production of by-products with small DNA sizes was also found.
Fig. 4 (not according to the present invention) shows the effect of exonuclease I treatment on the production of by-products in the TdT method when ddNTP was not used. Even in the case where exonuclease I treatment was carried out, reduction of small-sized by-products derived from free primers was not found. Moreover, elongation of the chain lengths of by-products due to the exonuclease I treatment was found.

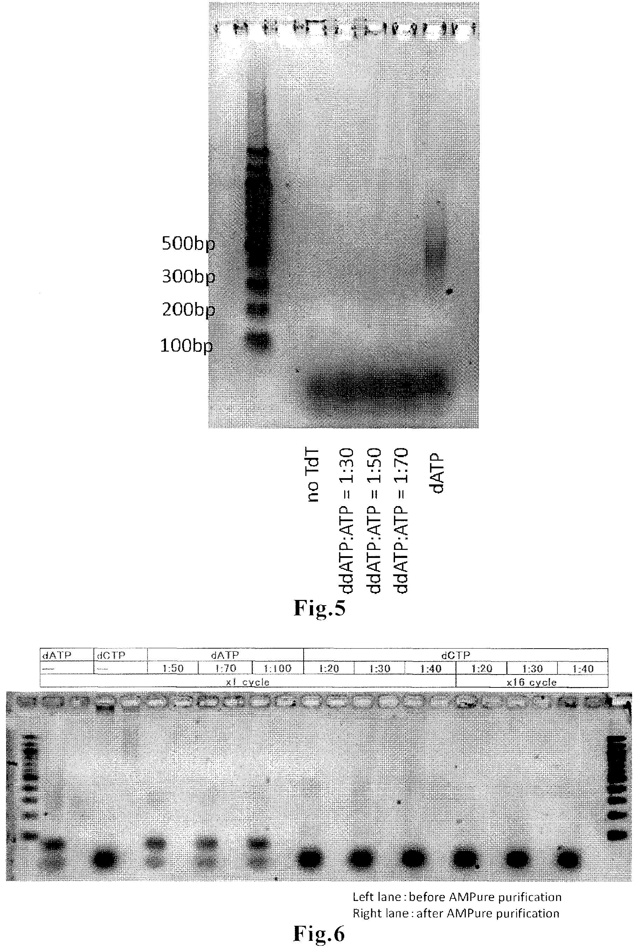

Fig. 5 (not according to the present invention) shows suppression of production of by-products by addition of ddATP in the TdT method. By adding a certain amount of ddATP, excessive elongation of by-products derived from free primers could be suppressed.
Fig. 6 shows suppression of production of by-products by addition of ddATP/ddCTP in the TdT method. By adding a certain amount of either ddATP or ddCTP in the TdT reaction, excessive elongation of the by-products derived from free primers could be suppressed. ddCTP showed better suppression of the lengths of short-chain by-products derived from free primers.

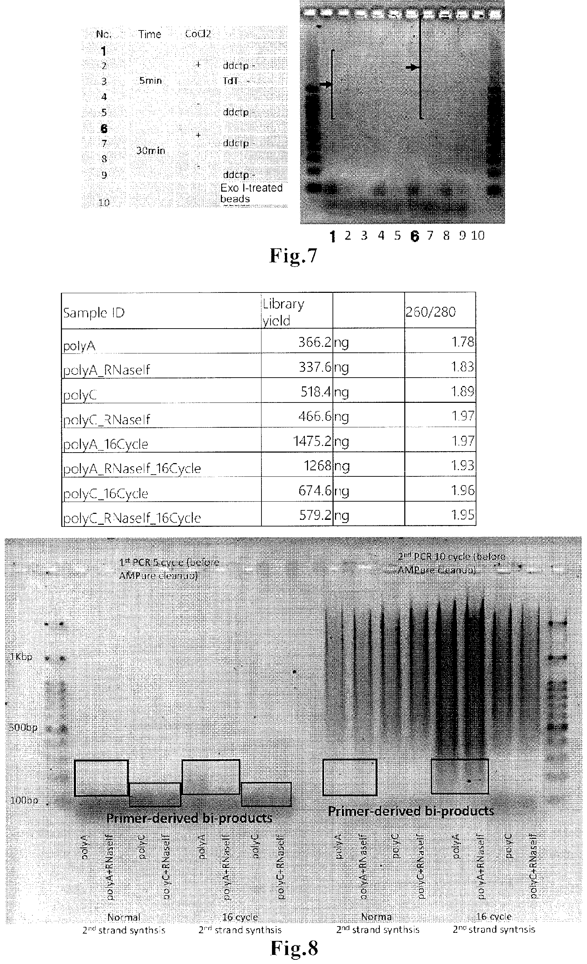

Fig. 7 shows the influence of the reaction time on the effect of inhibition of the production of by-products in the TdT method by addition of ddCTP. In the case where ddCTP was not added, elongation of by-products derived from free primers occurred when the reaction time was increased from 5 minutes to 30 minutes (Lane 2 and Lane 6). In contrast, in the case where ddCTP was added, the chain lengths of by-products derived from free primers remained short within a certain range even when the reaction time was increased from 5 minutes to 30 minutes. It can be seen that the influence of the reaction time length on the by-product elongation was reduced by addition of ddCTP.
Fig. 8 shows comparison of the cDNA amplification pattern in the TdT method with addition of ddATP/ddCTP, and the influences of RNase If and the multiple annealing method in the second-strand synthesis reaction. When multiple annealing was carried out in the second-strand synthesis, the cDNA yield increased in both the dATP method and the dCTP method. In contrast, RNase If had a negative influence. It can be seen that, although the ddATP method results in a high yield, the ddCTP method shows better suppression of elongation of by-products derived from free primers.

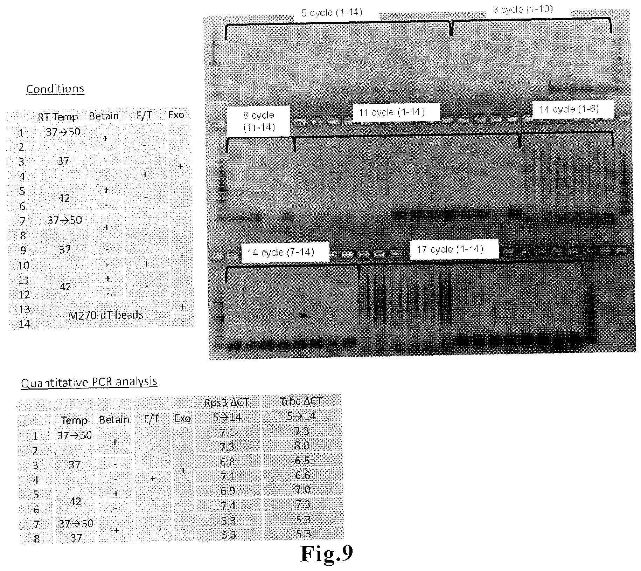

Fig. 9 shows a combined effect of the ddCTP-TdT method and exonuclease I treatment. By employing exonuclease I treatment in the ddCTP-TdT method, the by-products derived from free primers was largely decreased, and elongation of the by-products derived from free primers, as found when ddNTP was not used, was not found. Further, increases in the yield of amplified cDNA were found irrespective of the reverse transcription reaction conditions employed.

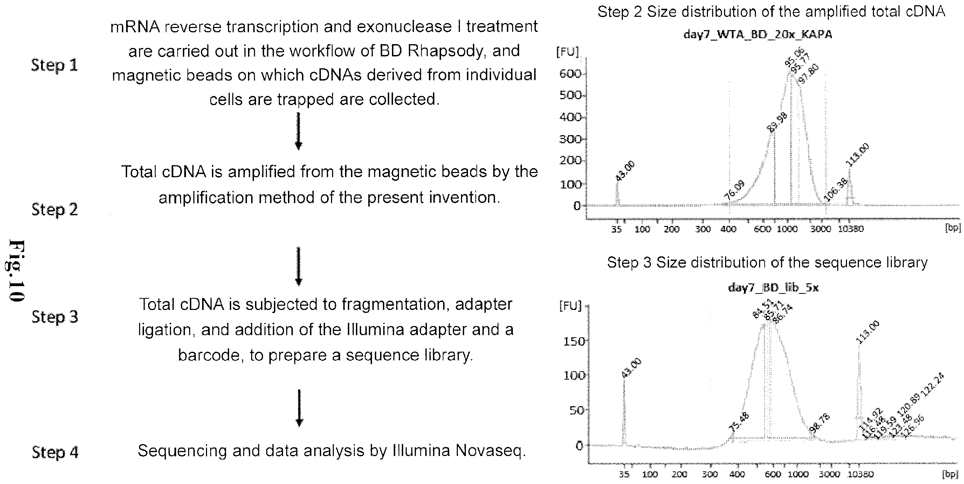

Fig. 10 is a scheme illustrating a method in which the total cDNA amplification from a bead solid-phase carrier according to the present invention is employed in combination with the BD Rhapsody System, which is an SCT analysis platform using a bead solid-phase carrier. The right panels show the DNA size distributions of the amplified cDNAs and the sequence library as analyzed using an Agilent Bioanalyzer.

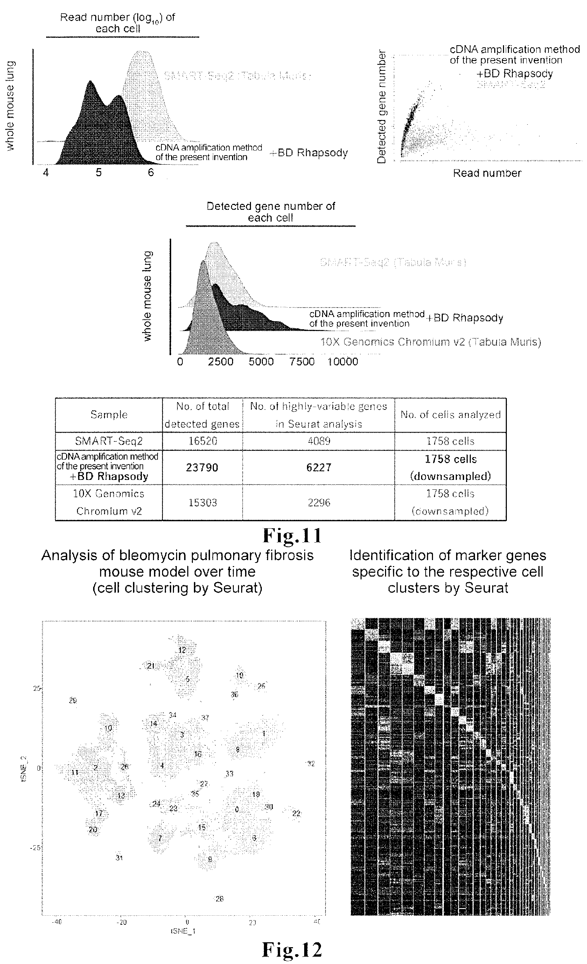

Fig. 11 shows an SCT analysis result obtained by the combination of the total cDNA amplification from a bead solid-phase carrier according to the method of the present invention and the BD Rhapsody System, and comparison of its performance with those of existing SCT analysis methods. The comparison was made on single cells prepared from mouse lung (data from the Tabula Muris consortium were used as the data from the existing platforms). Compared to the existing methods, the combination of the total cDNA amplification method according to the present invention and the BD Rhapsody System showed better results regarding any of the following: gene detection sensitivity per cell (compared with 10X Genomics Chromium and Smart-Seq2), the number of genes detected per identical sequence read (utilization efficiency; compared with Smart-Seq2), and resolution in the detection of differences in the gene expression between cells (evaluated based on the total number of detected genes and the number of highly-variable genes in Seurat analysis; bottom panel in Fig. 11).
Fig. 12 shows the result of analysis of a bleomycin-induced pulmonary fibrosis mouse model over time by an SCT analysis method employing the combination of the total cDNA amplification from a bead solid-phase carrier according to the method of the present invention and the BD Rhapsody System. Using data of mouse lung on Days 0, 3, 7, and 10 after the administration, analysis was carried out with Seurat software. Various cell populations of the lung were comprehensively identified, and marker genes characterizing the respective cell populations were also identified.
Fig. 13 shows the result of multiplex analysis of 14 specimens of CD45+ leukocytes derived from a tumor mouse model to which colon 26 was subcutaneously implanted, by an SCT analysis method employing the combination of the total cDNA amplification from a bead solid-phase carrier according to the method of the present invention, the BD Rhapsody System, and a DNA-labeled antibody. By using the method of the present invention for the total cDNA amplification, double-stranded DNAs derived from the DNA-labeled antibody for sample identification could be clearly separated from double-stranded DNAs derived from cDNAs. Further, the sources of the cells contained in the SCT data could be separated based on the sizes of the signals (numbers of reads) derived from the DNA-labeled antibody used for labeling. It was also found that a large number of genes could be detected uniformly among the specimens.

### MODE FOR CARRYING OUT THE INVENTION

In the present invention, A, T, G, and C constituting the base sequence of a target-capturing nucleic acid, an oligonucleic acid bound to a specific-binding molecule (the simple term "oligonucleic acid" as used hereinafter means an oligonucleic acid bound to a specific-binding molecule), a primer, an adapter, a nucleotide homopolymer, or the like include not only deoxyribonucleotides constituting DNA (A, T, G, and C of DNA), but also ribonucleotides constituting RNA (A, T, G, and C of RNA), and further, their corresponding nucleotide analogues (including bridged nucleic acids such as LNA, ENA, and PNA; and modified bases such as Super T (5-hydroxybutynl-2'-deoxyuridine), Super G (8-aza-7-deazaguanosine), deoxyinosine, 5-methyl dC, deoxyuridine, 2,6-diaminopurine, 2-aminopurine, and 2-Amino-dATP). Thus, the target-capturing nucleic acid (for example, any of the poly(T) portion, first adapter portion, barcode portion, oligonucleic acid-capturing region, and other portions), the nucleotide homopolymer, the second-strand-synthesizing primer (for example, any of the complementary homopolymer portion, second adapter portion, molecular barcode portion, and other portions), the oligonucleic acid (for example, any of the capture target region, common sequence, specific-binding-molecule-identification barcode portion, and other portions), and each primer used in the nucleic acid amplification step, may contain one or more (for example, about 1 to 15, about 1 to 12, or about 1 to several) monomers selected from ribonucleotides and nucleotide analogues.

In the present invention, a primer targeting a region X means a primer that specifically hybridizes with the region X or with the complementary strand thereof. Examples of the primer targeting a region X include primers comprising the same sequence as the region X or comprising the sequence complementary thereto. The term "primer (that is) set in a region X" has the same meaning, and means a primer that specifically hybridizes with the region X or with the complementary strand thereof. Examples of the primer set in a region X include primers comprising the same sequence as the region X or comprising the sequence complementary thereto.

The method for nucleic acid amplification of the present invention comprises a target nucleic acid-capturing step, a complementary-strand synthesis step, an exonuclease treatment step, an mRNA degradation step, a homopolymer addition step, a second-strand synthesis step, and a nucleic acid amplification step. Each step is described below with reference to Fig. 1 and Fig. 2 as appropriate.

### <Target Nucleic Acid-Capturing Step>

In the target nucleic acid-capturing step, a target nucleic acid is captured on a solid-phase carrier through a target-capturing nucleic acid bound to the solid-phase carrier (Step 1 in Fig. 1 and Fig. 2). The target nucleic acid is a nucleic acid comprising mRNA. The mRNA alone may be targeted, or a nucleic acid other than mRNA may also be targeted together. Examples of the target nucleic acid other than mRNA include an oligonucleic acid bound to a specific-binding molecule. Fig. 1 and Fig. 2 illustrate modes using a bead as the solid-phase carrier, wherein one mRNA molecule captured on one bead carrier is drawn for convenience. However, in practice, a large number of mRNA molecules are captured on one bead carrier. Step 1 in Fig. 1 illustrates a mode in which mRNA and oligonucleic acid are target nucleic acids, and one mRNA molecule captured on one bead carrier is also drawn in this mode for convenience. However, again, in practice, a large number of oligonucleic acid molecules are captured on one bead carrier.

The solid-phase carrier may be a bead, or a plate such as a slide glass. Examples of known single-cell analysis systems using a solid-phase bead include the Drop-seq method (Non-Patent Document 10 and WO 2016/040476 A1), the Nx1-seq method (WO 2015/166768 A1; Hashimoto et al. Scientific Reports 2017 Oct 27;7(1): 14225. doi: 10.1038/s41598-017-14676-3.), the Seq-well method (https://www.ncbi.nlm.nih.gov/pmc/articles/PMC5376227/), and the BD Rhapsody System. Examples of known analysis systems using a plate as the solid-phase carrier include the spatial transcriptomics (https://spatialtranscriptomics.com/products/), which uses an oligonucleic acid immobilized on a slide glass. The method for nucleic acid amplification of the present invention is applicable to any of these.

The material of the bead is not limited, and various materials used for nucleic acid-capturing carriers in nucleic acid analysis kits or for solid-phase particle carriers in immunoassay kits may be employed. Regarding the material of the bead, examples of the bead include beads made of an organic polymer, such as beads made of a resin, for example, polystyrene or polypropylene; beads made of a semiconductor, such as quantum dots (semiconductor nanoparticles) made of a semiconductor material, for example, cadmium selenide (CdSe), zinc sulfide (ZnS), cadmium sulfide (CdS), zinc selenide (ZnSe), or zinc oxide (ZnO); beads made of a metal such as gold; and polymer beads such as beads made of silica. Specific examples of the beads include beads made of a material such as cellulose, a cellulose derivative, an acrylic resin, glass, silica gel, polystyrene, gelatin, polyvinylpyrrolidone, a copolymer of vinyl and acrylamide, divinylbenzene crosslinked polystyrene or the like (see Merrifield Biochemistry 1964, 3,1385-1390), polyacrylamide, latex gel, polystyrene, dextran, rubber, silicon, plastic, nitrocellulose, cellulose, natural sponge, silica gel, glass, metal-plastic, cellulose, cross-linked dextran (including Sephadex (trade name)), or agarose gel (Sepharose (trade name)). Although the bead may be either a non-magnetic body or a magnetic body, a magnetic bead is preferably used since it can be more easily handled.

The material of the plate is also not limited, and, for example, various materials used for microarray substrates may be employed. Representative examples of the materials that may be used include glass, and also silicon and plastics. In cases where the solid-phase carrier is a plate, the target-capturing nucleic acid is usually immobilized on a plurality of compartments on the plate (typically immobilized such that aligned spots are formed).

The cell from which the mRNA is derived is not limited, and examples of the subject cell include various cells such as cells derived from mouse individuals, various cultured cells, cells derived from healthy individuals, cells derived from cancer patients, and cells derived from patients with various diseases. The cells derived from cancer patients may be cells derived from cancer patients receiving cancer immunotherapy. The cells derived from healthy individuals or patients may be cells collected from peripheral blood or a lesion (such as a tumor).

In cases where an oligonucleic acid and mRNA are used together as target nucleic acids, the specific-binding molecule to be labeled with the oligonucleic acid is preferably at least one kind of molecule that binds to a cell surface molecule directly, or indirectly through a specific-binding partner molecule.

Typical examples of the specific-binding molecule that directly binds to a cell surface molecule include an antibody against a cell surface molecule. The antibody is not limited as long as it has a binding capacity that allows the antibody to specifically bind to a cell surface molecule, which is an antigen, by antigen-antibody reaction. Antibody fragments (antigen-binding fragments) such as Fab, F(ab')₂, and scFv (single chain fragment of variable region; single-chain antibody) may also be used. The term "antibody fragment against a cell surface molecule" means an antibody fragment having a binding capacity that allows the antibody fragment to specifically bind to a cell surface molecule, which is an antigen, by antigen-antibody reaction. In cases where simultaneous analysis of mRNA expression and protein expression is carried out, an antibody/antibodies and/or antibody fragment(s) against one or more desired proteins may be used as a specific-binding molecule(s). In cases where simultaneous analysis of a plurality of samples (multiplex analysis) is carried out, an antibody/antibodies and/or antibody fragment(s) against a cell surface molecule(s) stably expressed on the cells to be analyzed may be used as a specific-binding molecule(s).

Preferred examples of the specific-binding molecule and the specific-binding partner molecule as the binding partner thereof include avidin-type molecules and biotin-type molecules, respectively. More specifically, as the specific-binding molecule, an avidin-type molecule such as avidin, streptavidin, or NeutrAvidin may be preferably used, and a biotin-type molecule such as biotin or desthiobiotin may be preferably used as the specific-binding partner molecule. In cases where a specific-binding molecule that indirectly binds to a cell surface molecule through a specific-binding partner molecule is used, cells may be treated with the specific-binding partner molecule to generally label cell surface molecules, and then the cells may be reacted with a specific-binding molecule labeled with an oligonucleic acid. For example, in cases where a hydrophilic biotin-labeling reagent such as Biotin-Sulfo-OSu or Biotin-SS-Sulfo-OSu is used, the reagent and the cells may be added to PBS, and the resulting mixture may be incubated at 4°C for about 10 to 30 minutes, to achieve non-specific biotin labeling of the cell surface molecules present on the cell surface. The indirect labeling method using a specific-binding molecule and its partner molecule is a method suitable for multiplex analysis since the method allows general, non-specific labeling of cell surface molecules without being influenced by the presence or absence of expression and the expression patterns, of the cell surface molecules.

Examples of the cell surface molecules include, but are not limited to, various cell surface molecules such as CD molecules, major histocompatibility antigens, and cell surface receptors.

The oligonucleic acid is typically a single-stranded DNA, and its chain length is usually about several ten bases to 150 bases, for example, about 60 bases to 100 bases. The oligonucleic acid comprises a capture target region to be captured by the target-capturing nucleic acid on its 3'-side (the side of the terminus not bound to the specific-binding molecule). When the region in the target-capturing nucleic acid that hybridizes with the capture target region is referred to as "oligonucleic acid-capturing region" for convenience, the capture target region has a sequence complementary to the oligonucleic acid-capturing region. Preferred examples of the capture target region include poly(A). However, the capture target region is not limited thereto, and may be composed of an arbitrary sequence that is not poly(A). A sequence whose melting temperature for the capturing region is higher than that of the poly(A) sequence and in which five or more consecutive G and/or C bases do not appear may be preferably employed as the arbitrary sequence that is not poly(A). The oligonucleic acid comprises, on its 5'-side (the side of the terminus bound to the specific-binding molecule), i.e., the 5'-upstream side of the capture target region, a common sequence in which a primer used in the second-strand synthesis or a primer used in the nucleic acid amplification step is set. Between the common sequence and the capture target region, a specific-binding-molecule-identification barcode for identification of the specific-binding molecule may be contained. By utilizing different kinds of specific-binding-molecule-identification barcodes in accordance with the number of samples, which sample each of the finally amplified nucleic acids is derived from can be identified.

In one mode of the target nucleic acid-capturing step, an mRNA sample is prepared from a cell sample such as a lesion tissue specimen or a cell suspension, and a solid-phase carrier to which a target-capturing nucleic acid is bound is brought into contact with the mRNA sample, to capture mRNA on the solid phase. In the preparation of the mRNA sample, first, total RNA may be extracted from the cell sample, and mRNA may then be extracted therefrom. Or, mRNA may be directly extracted from the cell sample without carrying out total RNA extraction.

In another mode of the target nucleic acid-capturing step, a single-cell transcriptome (SCT) analysis platform using a solid-phase bead is employed. In this mode, RNA is extracted from each single cell in a microwell or microdroplet, and brought into contact with a bead carrier to which a target-capturing nucleic acid is bound. By this, mRNA as a target nucleic acid is captured on the bead within the microwell or microdroplet. In the terms "microwell" and "microdroplet", the term "micro-" typically means that the volume is about 5 to 100 pl. Examples of common methods for SCT analysis platforms using solid-phase beads include the BD Rhapsody System by Beckton Dickinson (Patent Document 4), the Seq-well System (Non-Patent Document 11) and the Nx1-seq System (WO 2015/166768 A1; Hashimoto et al. Scientific Reports 2017 Oct 27; 7(1): 14225. doi: 10.1038/s41598-017-14676-3.), which use a plate having microwells; and the Drop-seq method, in which mRNA capture is carried out in a microdroplet (Non-Patent Document 10). In the present invention, these common methods may be preferably used.

In still another mode of the target nucleic acid-capturing step, a platform for the spatial transcriptome method is used. In this mode, a tissue sample is subjected to permeabilization treatment on a plate on which a target-capturing nucleic acid is immobilized, and mRNA derived from each cell of the tissue sample is captured with the capturing nucleic acid aligned and immobilized on the plate.

As the target-capturing nucleic acid, a nucleic acid comprising a poly(T) portion is preferably used. On the solid-phase carrier, a nucleic acid not comprising a poly(T) portion may also be immobilized as a target-capturing nucleic acid in addition to the nucleic acid comprising a poly(T) portion. In the method of the present invention, a nucleic acid for capturing mRNA is immobilized as the target-capturing nucleic acid on the solid-phase carrier, and a nucleic acid for capturing a target nucleic acid other than mRNA may also be additionally immobilized thereon. For example, as described above, a nucleic acid for capturing an oligonucleic acid may be additionally immobilized as a target-capturing nucleic acid on the solid-phase carrier. As described later in detail, in a mode in which an oligonucleic acid-labeled specific-binding molecule is used in the amplification method of the present invention, the target-capturing nucleic acid for capturing the mRNA and the target-capturing nucleic acid for capturing the oligonucleic acid, immobilized on the solid-phase carrier, may be identical to, or different from, each other.

The target-capturing nucleic acid may comprise a first adapter portion on the 5'-side, that is, the solid-phase carrier side, of the poly(T) portion. The portion represented as Universal 1 in Fig. 1 and Fig. 2 is the first adapter portion. The first adapter portion is a region in which one of the primers in the nucleic acid amplification step is set.

The target-capturing nucleic acid may comprise, between the first adapter portion and the poly(T) portion, a barcode portion for identifying each solid-phase carrier or each position on the solid-phase carrier. In cases where the solid-phase carrier is a bead, the barcode portion is a bead-identification barcode. In cases where the solid-phase carrier is a plate, the barcode portion is a compartment-identification barcode for identifying which compartment on the plate the target-capturing nucleic acid is immobilized on. In a mode in which such a barcode portion is included, the cDNA-comprising nucleic acid amplified in the nucleic acid amplification step comprises a barcode sequence derived from the identification barcode portion. The bead-identification barcode portions have the same sequence on the same bead, and have different sequences from one bead to another, by which cDNAs derived from mRNAs captured on the same bead can be distinguished from cDNAs derived from mRNAs captured on other beads. In SCT analysis, cDNAs derived from the same cell can be distinguished from cDNAs derived from other cells. An immobilized mRNA-capturing nucleic acid comprising such a bead-identification barcode portion can be prepared by a known method (see, for example, WO 2015/166768 A1). The compartment-identification barcode portions have the same sequence when they are in the same compartment (spot) on a plate, and have different sequences from one compartment (spot) to another, by which what kind of mRNA is expressed in which portion of the tissue can be understood when the sample is a tissue specimen.

### <Complementary-Strand Synthesis Step>

In the complementary-strand synthesis step, from the target nucleic acid captured on the solid-phase carrier, a DNA strand complementary thereto is synthesized. In cases where the target nucleic acid is mRNA, cDNA is synthesized by reverse transcription reaction. In cases where the target nucleic acid comprises a nucleic acid besides mRNA, such as an oligo DNA bound to a specific-binding molecule, reverse transcription reaction from mRNA to cDNA and complementary-strand synthesis reaction from the oligo DNA using DNA polymerase are carried out in the complementary-strand synthesis step. By this step, a DNA strand which is complementary to the target nucleic acid and which comprises the cDNA is synthesized from the target nucleic acid comprising mRNA captured on the solid-phase carrier. After the target nucleic acid-capturing step, the solid-phase carrier is collected, and reverse transcription reaction is carried out in the state where the target nucleic acid is captured on the solid-phase carrier (Step 1 in Fig. 1 and Fig. 2). The reverse transcription reaction from the target mRNA and the complementary-strand synthesis reaction from the target DNA, per se, may be carried out according to conventional methods. After the synthesis of the complementary strand, the solid-phase carrier is washed before proceeding to the following step.

### <Exonuclease Treatment Step>

In the exonuclease treatment step, target-capturing nucleic acid on the solid-phase carrier that has not captured the target nucleic acid is degraded and removed by exonuclease treatment (Step 2 in Fig. 1 and Fig. 2; "RT primers" in the figures means the target-capturing nucleic acid). Common examples of exonucleases that specifically degrade single-stranded DNA include exonuclease I and exonuclease T. In the present invention, at least one of such common exonucleases may be used. After the exonuclease treatment, the solid-phase carrier is washed before proceeding to the following step.

### <mRNA Degradation Step>

In the mRNA degradation step, the mRNA in the mRNA-cDNA hybrid bound on the solid-phase carrier is degraded by a ribonuclease (Step 3 in Fig. 1 and Fig. 2). A common ribonuclease such as RNase H may be used. After the degradation of the RNA, the solid-phase carrier may be washed and collected, followed by proceeding to the homopolymer addition step, or may be subjected as it is to the homopolymer addition step without washing. As described later, the mRNA degradation step and the homopolymer addition step may be carried out simultaneously.

### <Homopolymer Addition Step>

In the homopolymer addition step, reaction by terminal deoxynucleotidyl transferase (TdT) is carried out in the presence of dCTP or dGTP, and a chain-terminating nucleotide triphosphate (chain-terminating NTP), which is a chain terminating CTP or GTP, to add a nucleotide homopolymer which is poly(C) or poly(G) to the end of the DNA strand synthesized on the solid phase (Step 3 in Fig. 1 and Fig. 2; the figure illustrates a case with poly(C)). The chain-terminating NTP (chain-terminating CTP or chain-terminating GTP) is, as is well known in the art, a nucleotide modified or altered such that the OH group at the 3'-position of the nucleotide is not capable of forming a phosphoester bond with the 5'-phosphate portion of another nucleotide molecule. The chain-terminating NTP can also be said to be a nucleotide triphosphate having no OH group at the 3'-position comprising no OH group in the atomic group bound to the 3'-position). Specific examples of common chain-terminating NTPs that can also be used in the present invention include dideoxynucleotide triphosphates (ddNTPs) (ddCTP and ddGTP), ddNTP derivatives (typically ddNTPs whose 3'-position is modified with an atomic group having no OH group, such as ddNTPs whose 3'-position is modified with an azido group or an amino group, for example, 3'-azido-ddCTP, 3'-azido-ddGTP, 3'-amino-ddCTP, and 3'-amino-ddGTP), and 3'-deoxynucleotide triphosphates (3'-dNTPs) (3'-dCTP and 3'-dGTP). The chain-terminating NTP may be, for example, ddNTP, or ddNTP whose 3'-position is modified with an azido group, or may especially be ddNTP, although the chain-terminating NTP is not limited thereto. As the chain-terminating NTP and the substrate (dNTP) forming the homopolymer, those having the same base chain are preferably used. Because the minimum homopolymer length required for the second-strand synthesis reaction, the required length of the 3'-end homopolymer portion of the primer used for the second-strand synthesis, and the chain lengths of by-products derived from free primers can be desirably reduced, poly(C) addition or poly(G) addition is carried out by the combination of dCTP + chain-terminating CTP such as ddCTP, or dGTP + chain-terminating GTP such as ddGTP.

For example, in cases where ddNTP is used, the amount of the chain-terminating NTP added, in terms of the ratio to the dNTP (dCTP or dGTP), is ddNTP:dNTP = 1:15 to 1:40. The chain-terminating NTP is used at a ratio of 1: 15 to 1:40. The amounts of other chain-terminating NTPs is also set in accordance with this ratio. In cases where the TdT reaction is carried out in the absence of the chain-terminating NTP, the reaction time needs to be strictly controlled for controlling the chain length of the homopolymer in accordance with the lot-to-lot variation of the activity of the enzyme, and the differences in the amounts of the primers and substrate such as cDNA. In contrast, in the present invention, since the homopolymer addition reaction by TdT is carried out in the presence of the chain-terminating NTP, the chain-terminating NTP is stochastically incorporated to stop the homopolymer extension reaction, so that the acceptable degree of prolongation of the TdT reaction time can be largely increased.

The homopolymer addition reaction by TdT is generally carried out in the presence of a divalent cation. Examples of the cations that may be used include divalent metal cations such as Zn²⁺, Cu²⁺, Ni²⁺, Co²⁺, Mn²⁺, and Mg²⁺. For example, the cation may be, but is not limited to, Co²⁺ or Mn²⁺.

Since the addition of the chain-terminating NTP largely increases the acceptable degree of prolongation of the TdT reaction time, RNase H can be added to the TdT reaction liquid to carry out the mRNA degradation reaction and the homopolymer addition reaction simultaneously. Thus, in one mode of the present invention, the mRNA degradation step and the homopolymer addition step are carried out simultaneously.

### <Second-Strand Synthesis Step>

After the homopolymer addition reaction, the solid-phase carrier is washed before proceeding to the second-strand synthesis step. In the second-strand synthesis step, second-strand synthesis is carried out on the complementary DNA strand bound to the solid-phase carrier, using a second-strand-synthesizing primer that comprises a primer comprising a homopolymer portion complementary to the nucleotide homopolymer added to the end of the complementary DNA strand that comprises the cDNA (Step 4 in Fig. 1 and Fig. 2). In cases where not only the mRNA but also an oligonucleic acid bound to a specific-binding molecule is the target nucleic acid, the second-strand-synthesizing primer may comprise the above-described primer comprising a complementary homopolymer portion, and a primer comprising a partial region (the later-described common sequence) in the oligonucleic acid.

The complementary homopolymer portion is present in the 3'-end-side of the second-strand-synthesizing primer. The chain length of the complementary homopolymer portion is 6 to 13 bases. To the 3'-end of the complementary homopolymer portion, an anchor sequence of about 1 to 2 bases may be added. By the addition of the anchor sequence, the probability of annealing of the second-strand-synthesizing primer to the start point of the homopolymer tail added to the cDNA by TdT can be increased. As the anchor sequence, H or HN (H = A, T, or C; N = any base (A, T, C, or G)) may be used when the complementary homopolymer portion is poly(G); D or DN (D = A, T, or G; N = any base (A, T, C, or G)) may be used when the complementary homopolymer portion is poly(C).

In the second-strand synthesis step, the process of annealing-extension of the second-strand-synthesizing primer may be carried out a plurality of times by thermal cycling reaction. Further, after carrying out one cycle of second-strand synthesis, a polymerase having strand displacement activity such as Bst DNA polymerase may be used to carry out follow-up reaction. By such a treatment, the efficiency of the second-strand synthesis can be increased.

The second-strand-synthesizing primer may comprise a second adapter portion on the 5'-side of the complementary homopolymer portion. The portion represented as Universal 2 in Step 4 in Fig. 1, or the portion represented as trP1 in Step 4 in Fig. 2, is the second adapter portion. The second adapter portion serves a region in which one of the primers in the nucleic acid amplification step is set.

The second-strand-synthesizing primer may comprise a molecular barcode portion between the second adapter portion and the complementary homopolymer portion. The second-strand-synthesizing primer in this mode has a structure in which each portion is linked from the 5'-side in the following order: [second adapter]-[molecular barcode]-[complementary homopolymer]. The molecular barcode has a random base sequence. Among the DNA molecules amplified by the later nucleic acid amplification step, DNAs derived from the same double-stranded cDNA have the same molecular barcode, while DNAs derived from different double-stranded cDNAs have molecular barcodes that are different from each other. By counting only DNAs having different molecular barcodes, assumption of the number of cDNA molecules at the start, and correction of variation of the PCR amplification efficiency among different cDNAs are possible. Although the chain length of the molecular barcode is not limited, it is typically about 12 bases to 30 bases. To make the molecular barcode tolerant of sequencing errors, the molecular barcode may be configured such that a certain fixed sequence is inserted in the random base sequence.

### <Nucleic Acid Amplification Step>

After the synthesis of the second strand, the solid-phase carrier is washed or not washed and then the nucleic acid amplification step is carried out. In the nucleic acid amplification step, nucleic acid amplification reaction is carried out using, as a template, the DNA double strand synthesized on the solid-phase carrier (Step 5 and Step 6 in Fig. 1 and Fig. 2). Since this nucleic acid amplification reaction requires high-fidelity, it is preferred to use a polymerase generally known as a high-fidelity PCR enzyme. Various high-fidelity PCR enzymes are commercially available. In this step, a nucleic acid amplification reaction liquid can be added without washing the solid-phase carrier, to directly carry out the nucleic acid amplification reaction.

In a mode employing a first adapter and a second adapter, PCR may be carried out using a set of primers set in those adapters. In cases of SCT analysis (Fig. 1), mRNAs expressed in each cell are comprehensively reverse transcribed and amplified. Therefore, it is common to carry out total cDNA amplification with a primer set targeting the first and second adapters. Although the primer set in this case is typically a set of a primer composed of the first adapter sequence and a primer composed of the second adapter sequence, such primers that comprise modification with an arbitrary additional sequence, biotin, or the like in the 5'-side can also be used. In the example illustrated in Fig. 1, PCR is carried out twice using a primer composed of the first adapter sequence (Universal 1 primer) and a primer composed of the second adapter sequence (Universal 2 primer). This is one example of the cases where the nucleic acid amplification method of the present invention is used in SCT analysis, and the mode of use of the method in SCT analysis is not limited to this one example.

In cases where not only mRNA but also an oligonucleic acid bound to a specific-binding molecule is the target nucleic acid, a primer comprising a partial region of the oligonucleic acid (the later-described common sequence) is used together with a primer targeting the first adapter and a primer targeting the second adapter, to amplify cDNA and the oligonucleic acid simultaneously. By subjecting the resulting amplification product to size fractionation using magnetic beads or the like, the amplified cDNA and oligonucleic acid can be separated from each other, and can be independently amplified in the second PCR.

In cases where the present invention is carried out for preparation of a TCR variable-region library in TCR repertoire analysis (Fig. 2), cDNA regions encoding the TCR variable region may be amplified among the cDNA molecules synthesized on the solid-phase carrier. Thus, in this case, in the nucleic acid amplification step, a primer targeting the TCR constant region may be used instead of the primer targeting the first adapter. Or, a primer set targeting the first adapter and the second adapter may be used to carry out total cDNA amplification in the same manner as shown in Fig. 1, and then the TCR variable region cDNA may be amplified using the total cDNA amplification product as a template, and using a primer targeting the TCR constant region and a primer targeting the second adapter.

As examples of application of the nucleic acid amplification method of the present invention, the following illustrates an example of application to SCT analysis, an example of application to TCR repertoire analysis, and an example of application in combination with analysis using an oligonucleic acid-labeled specific-binding molecule. However, application of the nucleic acid amplification method of the present invention is not limited to the application to these analysis methods, and the method of the present invention can be applied to various techniques comprising a step of cDNA amplification from a solid-phase carrier such as a bead or plate.

### <Example of Application to SCT Analysis (Fig. 1)>

One example of application of the nucleic acid amplification method of the present invention to SCT analysis is described below based on Fig. 1. However, examples of the application to SCT analysis are not limited to this example.

Step 1 to Step 4 may be carried out in accordance with the above-described process from the target nucleic acid-capturing step to second-strand synthesis step. The second-strand-synthesizing primer does not need to comprise a molecular barcode portion between the second adapter portion and the complementary homopolymer portion.

The nucleic acid amplification step may be carried out as two amplification reactions (1st PCR and 2nd PCR). In both reactions, a primer targeting the first adapter and a primer targeting the second adapter are used. The 1st PCR (Step 5 in Fig. 1) is carried out as a PCR of several cycles, and size selection and purification of the 1st PCR amplification product is carried out. Subsequently, 2nd PCR is carried out to further amplify the total cDNA (Step 6 in Fig. 1), and then size selection and purification are carried out again, to obtain a total cDNA amplification product. For convenience in a later step in which fragments to be subjected to sequence analysis are collected, each primer used in the 2nd PCR may comprise a spacer such as biotin or amine bound to the 5'-end.

For subjecting the obtained total cDNA amplification product to sequencing with a next-generation sequencer, the product is processed by fragmentation, end repair, A-tailing, addition of a sequencing adapter, and the like. By these processes, a library of cDNA processed into a sequencing construct can be obtained. As the means of the fragmentation, ultrasonic or an enzyme may be used. For the preparation of the sequencing construct, commonly used reagents may be used. Representative examples of the enzyme method include those using a NEBNext UltraII FS kit manufactured by New England Biolabs, or a KAPA HyperPlus kit manufactured by KAPA Biosystems. Examples of ultrasonic fragmentation apparatuses that may be used include Covaris and Bioruptor. Other similar products may also be used. As the sequencing adapter, an appropriate adapter compatible with the next-generation sequencer employed may be used.

Sequencing of the library may be carried out using a sequencer that is generally called a next-generation sequencer. Specific examples of next-generation sequencers that may be preferably used include the Novaseq 6000 system, the Hiseq system, the Nextseq system, and the MiSeq system, manufactured by Illumina, Inc.; and the Ion Proton system and the Ion S5/Ion S5 XL system, manufactured by Thermo Fisher Scientific Inc.

### <Example of Application to TCR Repertoire Analysis (Fig. 2)>

One example of application of the nucleic acid amplification method of the present invention to TCR repertoire analysis is described below based on Fig. 2. However, examples of the application to TCR repertoire analysis are not limited to this example.

Step1 to Step 4 may be carried out in accordance with the process from the target nucleic acid-capturing step to the second-strand synthesis step except that a sequencing adapter suitable for the next-generation sequencer employed for the sequencing analysis is used as the second adapter (trP1 in Step 4 in Fig. 2). A second-strand-synthesizing primer comprising a molecular barcode portion is preferably used.

The nucleic acid amplification step may be carried out as two amplification reactions (1st PCR and 2nd PCR). In the 1st PCR, a primer that is set in the TCR constant region (TRAc and/or TRBc external primer(s) in Step 5 in Fig. 2) and a primer targeting the second adapter (trP1 primer in Fig. 2) are used. In cases where the subject to be analyzed by the TCR repertoire analysis is the β-chain, a primer targeting the β-chain constant region (TRBc external primer) and a primer targeting the second adapter may be used. In cases where the subject to be analyzed is the α-chain, a primer targeting the α-chain constant region (TRAc external primer) may be used instead of the primer targeting the β-chain constant region. By mixing a primer targeting the β-chain constant region and a primer targeting the α-chain constant region together (TRAc and/or TRBc external primers), and using a set of the mixed primers and a primer targeting the second adapter, both the β-chain variable region and the α-chain variable region can be amplified.

The 1st PCR in Step 5 may be carried out by nested PCR. In this case, for both the first and second PCRs in the nested PCR, the primer to be used in combination with the primer targeting the second adapter may be set within the constant region. In such a case where one of the primers is the same between the first and second PCRs, the nested PCR is sometimes referred to as semi-nested PCR. Also in this step, by using the set of the primer set in the adapter and the primers set in the constant region, cDNAs of TCRs can be amplified without suffering from a PCR bias.

After carrying out size selection and purification of the 1st PCR amplification product, 2nd PCR is carried out (Step 6 in Fig. 2). In the 2nd PCR, a primer composed of the second adapter sequence (trP1 primer) and a primer targeting the constant region on the TCR cDNA fragments (A-BC-TRC primer) are used. The A-BC-TRC primer, which targets the TCR constant region, has the sequence of a second sequencing adapter (lonA) at the 5'-end. By such 2nd PCR, a TCR variable-region library composed of DNA fragments having sizes of about 500 to 600 bp to which sequencing adapters are added to both ends can be obtained. The A-BC-TRC primer, which targets the TCR constant region, used in the 2nd PCR has a molecular barcode sequence (BC) between the sequence of the second sequencing adapter (lonA) and the TCR constant-region-targeting sequence. The same molecular barcode is used for the same library prepared from the same sample, and different molecular barcodes are used for different libraries. By this, the library from which a certain TCR variable-region sequence is derived can be identified after large-scale sequence analysis by the next-generation sequencer.

Sequencing of the TCR variable-region library may be carried out using a sequencer that is generally called a next-generation sequencer. Specific examples of next-generation sequencers that may be preferably used include the Ion S5/Ion S5 XL system, manufactured by Thermo Fisher Scientific Inc.; and the MiSeq system and the Novaseq 6000 system, manufactured by Illumina, Inc.

### <Example of Use in Combination with Analysis Using Oligonucleic Acid-Labeled Specific-Binding Molecule (Fig. 1)>

One example of a case where an oligonucleic acid-labeled specific-binding molecule and the amplification method of the present invention are used in combination is described below based on Fig. 1. Although Fig. 1 illustrates a case where the amplification method of the present invention is used for SCT analysis, examples of combined use of an oligonucleic acid-labeled specific-binding molecule and the amplification method of the present invention are not limited thereto. The amplification method of the present invention may be used in combination not only with TCR repertoire analysis, but also with various techniques that comprise a step of cDNA amplification from a solid-phase carrier such as a bead or plate.

In cases where an oligonucleic acid-labeled specific-binding molecule is used in combination, mRNA and an oligonucleic acid are the target nucleic acids. The target-capturing nucleic acid immobilized on the solid-phase carrier comprises a nucleic acid comprising an mRNA-capturing region for capturing mRNA and a nucleic acid comprising an oligonucleic acid-capturing region for capturing the oligonucleic acid. These nucleic acids may be either the same or different. In cases where the capture target region the oligonucleic acid has is poly(A), poly(T) can be used as the oligonucleic acid-capturing region the target-capturing nucleic acid has. In such cases, both target nucleic acids can be captured by a single kind of target-capturing nucleic acid comprising a poly(T) portion. In cases where the oligonucleic acid has an arbitrary sequence that is not poly(A) as the capture target region, a nucleic acid comprising an oligonucleic acid-capturing region whose sequence is complementary to the arbitrary sequence can be used as a target-capturing nucleic acid for capturing the oligonucleic acid to which the specific-binding molecule is bound. Thus, in such cases, the two kinds of nucleic acids, that is, a target-capturing nucleic acid comprising poly(T) as an mRNA-capturing region, and a target-capturing nucleic acid comprising a sequence complementary to the arbitrary sequence as an oligonucleic acid-capturing region, are immobilized on the bead carrier.

Before the target nucleic acid-capturing step in Step 1, a step of bringing a sample comprising a cell into contact with an oligonucleic acid-labeled specific-binding molecule, to bind the oligonucleic acid-labeled specific-binding molecule to the cell surface, and a step of lysing the cell, are carried out. In cases of indirect labeling using a specific-binding molecule and a partner molecule thereof, first, as described above, the cell may be treated with the specific-binding partner molecule (such as a biotin-type molecule), to generally and non-specifically label the cell surface molecules, and then the sample comprising this cell may be brought into contact with the specific-binding molecule (such as an avidin-type molecule) labeled with an oligonucleic acid, to allow the reaction. Thus, in the cases of indirect labeling, a step of bringing the specific-binding partner molecule into contact with the cell sample, to bind the specific-binding partner molecule to the cell surface molecules present on the cell surface, may be first carried out, and then a step of binding the oligonucleic acid-labeled specific-binding molecule to the cell surface may be carried out. In cases where the present invention is combined with SCT analysis, a cell reacted with the oligonucleic acid-labeled specific-binding molecule may be loaded on a platform for the SCT analysis, and the cell may be lysed in a microwell or microdroplet.

In Step 1, mRNA and the oligonucleic acid are captured on the bead carrier, and cDNA synthesis from the mRNA by reverse transcription reaction and synthesis of the complementary strand from the oligonucleic acid by DNA polymerase are carried out.

Step 2 may be carried out in accordance with the exonuclease treatment step described above.

Step 3 may be carried out in accordance with the mRNA degradation step and the homopolymer addition step described above. Homopolymers are added to the ends of both the cDNA reverse-transcribed from the mRNA and the complementary DNA strand synthesized from the oligonucleic acid. By the mRNA degradation, the cDNA on the bead carrier becomes a single-stranded state, whereas the oligonucleic acid is captured on the bead carrier in the state where a double strand with its complementary strand is formed. This double strand may be heat-denatured before the homopolymer addition reaction, to dissociate the oligonucleic acid strand to which the specific-binding molecule is bound. Or, the double strand may be heat-denatured after the homopolymer addition, to cause the dissociation.

In Step 4, as the second-strand-synthesizing primer, a primer comprising the second adapter portion on the 5'-side of the complementary homopolymer portion, and a primer comprising the common sequence, are used. The former is the primer for synthesizing the second strand of the cDNA synthesized from the mRNA, and the latter is the primer for synthesizing the second strand of the complementary strand synthesized from the oligonucleic acid. In cases where the present invention is combined with SCT analysis, the former second-strand-synthesizing primer does not need to comprise a molecular barcode portion between the second adapter portion and the complementary homopolymer portion. In cases where the present invention is combined with TCR repertoire analysis, a second-strand-synthesizing primer comprising a molecular barcode is preferably used.

In Step 5, the 1st PCR in the nucleic acid amplification step is carried out using a primer targeting the first adapter portion, a primer targeting the second adapter portion, and a primer comprising the common sequence. By a set of the primer targeting the first adapter and the primer targeting the second adapter, the cDNA synthesized from the mRNA is amplified. By a set of the primer targeting the first adapter and the primer comprising the common sequence, the oligonucleic acid is amplified.

Or, in Step 5, a primer (which is referred to as primer X, for convenience) targeting a desired region in the cDNA may be further used in combination. In cases where the present invention is combined with TCR repertoire analysis, a primer targeting the TCR constant region is used as the primer X. By a set of the primer X and the primer targeting the second adapter, the TCR variable region is amplified. By a set of the primer targeting the first adapter and the primer comprising the common sequence, the complementary strand synthesized from the oligonucleic acid is amplified.

The amplification product obtained by the 1st PCR is subjected to size fractionation using magnetic beads or the like, to separate the amplified cDNA and oligonucleic acid from each other. In the 2nd PCR in Step 6, the cDNA and the oligonucleic acid are separately and independently amplified.

### EXAMPLES

The present invention is described below more concretely by way of Examples. However, the present invention is not limited to the following Examples.

### 1. Comparison between SMART Method and Conventional TdT Method (Fig. 3; not according to the present invention)

A single-cell transcriptome library trapped on solid-phase beads, prepared by the Nx1-seq method (WO 2015/166768 A1; Hashimoto et al. Scientific Reports 2017 Oct 27;7(1): 14225. doi: 10.1038/s41598-017-14676-3.), was amplified by the SMART method and the conventional TdT method (using neither ddNTP nor exonuclease I). The sequences of the primers used are shown in Table 1 below.

For the preparation of the library, a nucleic acid (LibA-BC-linker-UMI-dT27VN) comprising a universal adapter portion, a bead-identification barcode portion, and a poly(T) portion was used as a target-capturing nucleic acid. The target-capturing nucleic acid was immobilized on non-magnetic beads, and these beads were filled one by one into wells of a microwell plate. Cells derived from a mouse lung single-cell suspension were loaded such that most of the wells contained one or zero cell, and mRNA molecules derived from each cell were captured as a target nucleic acid on each bead, to provide a single-cell transcriptome library.

In the SMART method, the above-described mRNA-capturing beads were subjected to reverse transcription using a reverse transcription reaction liquid (comprising Superscript II reverse transcriptase, Betain, First-strand buffer, RNase Inhibitor, MgClz, and Biosg-LibB-TSO primer) prepared according to the method described in Non-Patent Document 4, and the cDNA was prepared into a double strand, followed by total cDNA amplification by PCR using primers (Biosg-LibA-primer and Biosg-LibB-primer) for the LibB/LibA sequences added to both ends of the cDNA.

In the conventional TdT method, reverse transcription was carried out with Superscript IV, and the mRNA was degraded by RNase H, followed by washing the beads and carrying out TdT reaction for 50 seconds using dATP and CoCh. The reaction was immediately stopped using EDTA, and TdT was heat-inactivated, followed by washing the beads. Second-strand synthesis reaction was then carried out by a single time of annealing using LibB-dT25VN-primer. After washing the beads, total cDNA was amplified in the same manner as in the SMART method by PCR using the primers (Biosg-LibA-primer and Biosg-LibB-primer) for the LibB/LibA sequences added to both ends of the cDNA.

The results are shown in Fig. 3. In the conventional TdT method, a larger amount of cDNA was obtained compared to the SMART method, but contamination with by-products derived from the mRNA-capturing nucleic acid was found in the small-size region.

**[Table 1]**

| Primer name | Sequence | SEQ ID NO |
|---|---|---|
| LibA-BC-linker-UMI-dT27VN | CTATGCGCCTTGCCAGCCCGCTCAGACNNNNNNNNN NNNGATGGCGCGAGGGAGGCGATACGNNNNNNNNNTT TTTTTTTTTTTTTTTTTTTTTTTTTTVN | 1 |
| Biosg-LibB-TSO | Biosg/CTATGCGCCTTGCCAGCCCGCTCAGACGGG | 2 |
| LibB-dT25VN-primer | CGTATCGCCTCCCTCGCGCCAAGTCTGTTTTTTTTTTT TTTTTTTTTTTTVN | 3 |
| Biosg-LibA-primer | Biosg/CTATGCGCCTTGCCAGCCCGCTCAGAC | 4 |
| Biosg-LibB-primer | Biosg/CGTATCGCCTCCCTCGCGCCATCAGAC | 5 |

| | | |
|---|---|---|
| Biosg: Biotin modification The underlined GGG of Biosg-LibB-TSO was designed as follows: [RNA guanine]-[RNA guanine]-[LNA guanine]. | | |

### 2. Influence of Exonuclease I Treatment on TdT Method (Fig. 4; not according to the present invention)

The solid-phase beads to be used in the Nx1-seq method were treated with exonuclease I without carrying out mRNA trapping and cDNA synthesis. TdT reaction was carried out, and the pattern of production of by-products was compared. In spite of the fact that the exonuclease I treatment was carried out, reduction in the amount of by-products was not observed, and the lengths of the by-products were found to be even longer (Fig. 4).

### 3. Influence of ddNTP on By-Product Production in TdT Method (Figs. 5, 6, and 7)

As a target (mRNA)-capturing nucleic acid, BioEcoP-dT25-adapter was immobilized on Dynabeads M270 streptavidin (Thermo Fisher Scientific). Using the beads, the inhibitory effect of ddNTP on the production of by-products in the TdT reaction was studied. For the second-strand synthesis, 5'WTA-dT25 primer or 5'WTA-dG9 primer was used. The cDNA amplification was carried out using 5'WTA primer and 3'WTA primer. In the TdT reaction, ddATP was added when dATP was used (poly(A) tail addition; not according to the present invention), or ddCTP was added when dCTP was used (poly(C) tail addition), at a ratio of ddNTP:dNTP = 1:20 to 1:100, and the TdT reaction was carried out for 20 minutes (the ratio of the addition is described in the figures). It was found that, even when the TdT reaction time was excessive, use of either ddATP or ddCTP resulted in complete suppression of elongation of by-products (Figs. 5 and 6). For the cases where ddCTP was used, comparison was made between the case where the TdT reaction was carried out for 5 minutes and the case where the TdT reaction was carried out for 30 minutes. In the groups in which ddCTP was not added, production of primer-derived by-products of more than 1 kbp was found at a reaction time of 5 minutes, and the by-products were found to be even longer at 30 minutes. In contrast, in the groups in which ddCTP was added, the lengths of the primer-derived by-products remained about 100 bp at both reaction times of 5 minutes and 30 minutes, exhibiting no remarkable elongation (Fig. 7). Further, in the group in which Co²⁺ was added, synthesis of the second strand was more efficient than in the group in which Mg²⁺ was added, and the by-products completely disappeared by Exo I treatment (lane 10 in Fig. 7).

Excessive elongation of primer-derived by-products makes the size of the by-products close to the size of the cDNA amplification products of interest, which makes it difficult to separate the by-products and the cDNA amplification products from each other, to result in inhibition of cDNA amplification, which is problematic. As revealed by the present experiment, when the TdT reaction is carried out with addition of ddNTP, excessive elongation of primer-derived by-products does not occur even if the TdT reaction is excessively carried out due to, for example, a lot-to-lot variation of the enzyme or a difference in the skill of the experimenter, and thus inhibition of the cDNA amplification due to elongated by-products can be prevented.

**[Table 2]**

| Primer name | Sequence | SEQ ID NO |
|---|---|---|
| BioEcoP-dT25-adapter | 5BiotinTEG/AAGCAGTGGTATCAACGCAGAGTACTTTTTTTTTTTTTTT TTTTTTTTTT | 6 |
| 5'WTA-dT25 | GCGGCTGAAGACGGCCTATGTTTTTTTTTTTTTTTTTTTTTTTTT | 7 |
| 5'WTA-dG9 | GCGGCTGAAGACGGCCTATGTGGGGGGGGG | 8 |
| 5'WTA | GCGGCTGAAGACGGCCTATGT | 9 |
| 3'WTA | AAGCAGTGGTATCAACGCAGAGT | 10 |

| | | |
|---|---|---|
| 5BiotinTEG: 5' biotin-triethylene glycol modification | | |

### 4. Influences of cDNA Amplification by ddNTP Method and Plurality of Times of Annealing in Second-Strand Synthesis Reaction (Fig. 8)

Using the beads on which the target-capturing nucleic acid is immobilized, prepared in Section 3 above, mRNA as a target nucleic acid was captured and reverse-transcribed, and cDNA was amplified by the ddNTP-TdT method (a method in which TdT reaction is carried out in the presence of ddNTP). As the ddNTP method, the ddATP method, in which a poly(A) tail is added (not according to the present invention), or the ddCTP method, in which a poly(C) tail is added, was carried out. In both the ddATP method and the ddCTP method, the cDNA yield was found to be increased by carrying out a plurality of times of primer annealing reaction in the second-strand synthesis reaction. However, in the case of the ddATP method, production of a small amount of by-products having small sizes derived from the mRNA-capturing nucleic acid was found. It was thus found that, for achieving more robust cDNA amplification while maintaining a high yield, the ddCTP method is superior to the ddATP method. On the other hand, addition of RNase If, which is a ribonuclease for single-stranded RNA, to the reaction system resulted in a slight decrease in the amount of cDNA.

### 5. Effect of Combined Use of cDNA Amplification by ddCTP Method and Exonuclease I (Fig. 9)

Using the beads on which the target-capturing nucleic acid is immobilized, prepared in Section 3 above, mRNA as a target nucleic acid was captured and reverse-transcribed under various conditions. After the reverse transcription, the beads were divided into two halves, and one half was subjected to exonuclease I treatment. Thereafter, total cDNA was amplified by the ddCTP-TdT method. As a result, it was found that, by the exonuclease I treatment, by-products derived from the target-capturing nucleic acid decreased, and the cDNA yield increased about 2- to 4-fold.

### 6. Effect of Application of Present Invention to Single-Cell Transcriptome Analysis Platform Using Solid-Phase Magnetic Beads

The method for robust, highly sensitive amplification of cDNA immobilized on beads, according to the present invention is widely applicable, irrespective of the platform employed, to systems using a solid-phase carrier, such as comprehensive single-cell RNA-seq methods using a solid-phase bead, including the Drop-seq method (Non-Patent Document 10 and WO 2016/040476 A1), the Nx1-seq method (WO 2015/166768 A1; Hashimoto et al. Scientific Reports 2017 Oct 27;7(1): 14225. doi: 10.1038/s41598-017-14676-3.), the Seq-well method
(https://www.ncbi.nlm.nih.gov/pmc/articles/PMC5376227/), and the BD Rhapsody System; and the spatial transcriptome method (spatial transcriptomics AB; https://spatialtranscriptomics.com/products/), wherein a solid-phase oligo on a slide glass, which is a solid-phase carrier other than a bead, is used. For testing the usefulness of the present invention in single-cell transcriptome analysis, the BD Rhapsody System was used as an example, and the present amplification method was applied thereto to carry out single-cell transcriptome analysis of total cells in lungs of mice in the steady state and lungs of bleomycin-injured mice (Day 3, Day 7, and Day 10 after administration).

A lung of an 8-week-old mouse was removed after perfusion, minced using a razor blade, and enzymatically digested using Liberase TM and DNase I, to obtain a single-cell suspension of the whole lung. Dead cells and erythrocytes were removed by density gradient centrifugation using Percoll, and the cells were counted using a flow cytometer. The cells and beads were loaded on a microwell cartridge at appropriate densities according to the specification of Rhapsody. The cells were then lysed, and mRNA derived from each single cell was trapped on a single bead (comprising the Rhapsody universal adapter as an mRNA-capturing nucleic acid) (mRNA-capturing step). Reverse transcription reaction was carried out as recommended by the manufacturer (cDNA synthesis step), and then exonuclease I treatment was carried out to remove unreacted mRNA-capturing nucleic acid on the beads (exonuclease treatment step). The Rhapsody universal adapter immobilized on the beads is DNA having the following structure, wherein 96 kinds of known unique sequences consisting of 9 bases are employed for each of CLS1 (cell label section 1) to CLS3, so that a total of 288 known unique sequences constitute a total of about 900,000 types of bead-identification barcode portions. In SEQ ID NO: 11 of SEQUENCE LISTING, CLS1 to CLS3 are represented as NNNNNNNNN.
<Structure of Rhapsody Universal Adapter>

ACACGACGCTCTTCCGATCT-(CLS1)-ACTGGCCTGCGA-(CLS2)-
GGTAGCGGTGACA-(CLS3)-NNNNNNNNTTTTTTTTTTTTTTTTTT (SEQ ID
NO:11)

After the exonuclease I treatment, half of the beads (equivalent to about 3000 to 4000 cells) were used to amplify total cDNA according to the amplification method of the present invention. The mRNA degradation step and the homopolymer addition step were allowed to proceed simultaneously by addition of RNase Hand TdT to the reaction liquid. In the homopolymer addition step, TdT reaction was carried out using ddCTP in an amount of ddCTP:dCTP = 1:20 to 1:40 in a reaction liquid comprising Tris-HCl, (NH₄)₂SO₄, KCl, K-phosphate, MgSO₄, CoCl₂, Triton X-100, and glycerol, to add poly(C) tails to the cDNA ends on the magnetic beads such that their lengths were limited. After washing the beads, LibA-dG9 primer and KAPA HiFi Hotstart Readymix, which is a high-fidelity PCR enzyme, were used to carry out the second-strand synthesis reaction. The number of times of annealing performed was 16. After washing the beads, 1st PCR was carried out using NH2-LibA primer and NH2-universal primer, and KAPA HiFi Hotstart Readymix. After two times of purification using AmPure XP beads at 0.65-fold concentration, 2nd PCR was carried out using NH2-LibA primer and NH2-universal primer, and KAPA HiFi Hotstart Readymix. The DNA concentration and the size distribution were investigated using an Agilent Bioanalyzer. As a result, it was found that about 1 µg of cDNA with a peak of 1 to 1.5 kb was obtained by a total of 17 cycles of PCR (Fig. 10). The sequences of the primers used in each step are as shown in Table 3. Using a NEBNext UltraII FS kit according to the protocol for the kit, the obtained cDNA was fragmented using a NEBNext UltraII FS kit according to the protocol for the kit, followed by addition of an adapter, and addition of an illumina sequencing adapter and a barcode by PCR, to construct a library for sequence analysis (Fig. 10). The obtained library was quantified using a KAPA Library quantification kit for Illumina, and its size distribution was investigated using an Agilent Bioanalyzer. Sequencing was carried out using Illumina Novaseq 6000. The sequences of the adapters and primers used for the preparation of the library for sequence analysis are as shown in Table 4.

**[Table 3]**

| Primer name | Sequence | SEQ ID NO |
|---|---|---|
| LibA-dG9 | CTATGCGCCTTGCCAGCCCGCTCAGACTGGGGGGGGG | 12 |
| NH2-LibA | /5AmMC6/CTATGCGCCTTGCCAGCCCGCTCAGACT | 13 |
| NH2-Universal | /5AmMC6/ACACTCTTTCCCTACACGACGCTCTTCCGATCT | 14 |

| | | |
|---|---|---|
| 5AmMC6: 5' -(CH₂)₆-NH₂ modification | | |

**[Table 4]**

| Primer name | Sequence | SEQ ID NO |
|---|---|---|
| Illumina adapter | Phos-GATCGGAAGAGCACACGTCTGAACTCCAGTCAC-/AmMO/ T*CTAGCCTTCTCG | 15 |
| | | 16 |
| ILMN_I5_S531 | AATGATACGGCGACCACCGAGATCTACACCGGCATTAACACTCTTTCCCTACACGA*C | 17 |
| ILMN_I5_S589 | AATGATACGGCGACCACCGAGATCTACACCACGCAATACACTCTTTCCCTACACGA*C | 18 |
| ILMN_I5_S587 | AATGATACGGCGACCACCGAGATCTACACGGAATGTCACACTCTTTCCCTACACGA*C | 19 |
| ILMN_I5_S503 | AATGATACGGCGACCACCGAGATCTACACTGGTGAAGACACTCTTTCCCTACACGA*C | 20 |
| ILMN_I7_S771 (UID) | CAAGCAGAAGACGGCATACGAGATTGACTGACGTGACTGGAGTTCAGACGTG*T | 21 |
| ILMN_I7_S779 (UID) | CAAGCAGAAGACGGCATACGAGATATGGAAGGGTGACTGGAGTTCAGACGTG*T | 22 |
| ILMN_I7_S788 (UID) | CAAGCAGAAGACGGCATACGAGATGTGTTCCTGTGACTGGAGTTCAGACGTG*T | 23 |
| ILMN_I7_S739 (UID) | CAAGCAGAAGACGGCATACGAGATGCTGTAAGGTGACTGGAGTTCAGACGTG*T | 24 |

| | | |
|---|---|---|
| * represents a phosphorothioate bond. Illumina adapter is an adapter prepared by annealing of two complementary primers. Phos: 5' phosphorylation. 3AmMO: 3' NH₂ modification The primers were used in the following combinations. ILMN_I5_S531 and ILMN_I7_S771 ILMN_I5_S589 and ILMN_I7_S779 ILMN_I5_S587 and ILMN_I7_S788 ILMN_I5_S5O3 and ILMN_I7_S739 | | |

The single-cell transcriptome data obtained were compared with analysis data of mouse lung cells in the steady state obtained by existing SCT analysis methods (the Smart-seq2 method, and the Chromium system by 10X Genomics). As the data by the existing analysis methods, data from the Tabula Muris consortium were used. As a result, it was found that the combination of BD Rhapsody and the cDNA amplification method of the present invention is superior to the two existing methods regarding the total number of genes detected and the number of genes detected per cell in spite of the fact that the amount of sequences was 1/10 with respect to the Smart-seq2 method (Fig. 11). Each type of single-cell transcriptome data was evaluated in terms of the resolution at which the differences in gene expression among the cells could be detected, based on the number of highly-variable genes (genes whose expression largely varied among cells) as detected by Seurat software. As a result, the combination of BD Rhapsody and the cDNA amplification method of the present invention was found to have a resolution higher than those of the two existing methods (Fig. 11). Further, Seurat software was used to analyze the data collected over time (on Day 0, Day 3, Day 7, and Day 10) including those of the lungs of the bleomycin-injured mice. As a result, cell populations having various properties were comprehensively identified from the lungs, and marker genes expressed specifically in the respective cell populations were also identified (Fig. 12). By this, usefulness of the amplification method of the present invention in single-cell transcriptome analysis was demonstrated.

### 7. Effect of Present Invention in Single-Cell Transcriptome Analysis Using Oligonucleic Acid-Labeled Antibody in Combination

As an example of single-cell transcriptome analysis using an oligonucleic acid-labeled antibody in combination, an analysis example of CD45-positive leukocytes derived from a mouse tumor model to which colon 26 was subcutaneously implanted is described below. As the oligonucleic acid-labeled antibody, DNA-labeled antibodies in which an antibody (a mixture of an anti-CD45 antibody and an anti-MHC class I antibody; trade name, Biolegend Totalseq Hashtag-A: https://www.biolegend.com/en-us/totalseq) was labeled with either one of 14 kinds of different single-stranded DNAs was used. The label DNAs are DNAs having the following structure, wherein 14 kinds of known unique sequences consisting of 15 bases are employed for the barcode portion. In SEQ ID NO:25 of SEQUENCE LISTING, the barcode portion is represented as NNNNNNNNNNNNNNN. The DNAs have poly(A) as a capture target region, and hence can be captured, similarly to mRNA, by a target-capturing nucleic acid having a poly(T) portion.

### <Label DNA Structure of Biolegend Hashtag Totalseq-A>

GTGACTGGAGTTCAGACGTGTGCTCTTCCGATCT-

NNNNNNNNNNNNNNN-

BAAAAAAAAAAAAAAAAAAAAAAAAAAA*A*A (SEQ ID NO:25);

* represents a phosphorothioate bond.

The subcutaneous tumor was removed from each mouse, and enzymatically digested with Liberase TM and DNase I, to prepare a single-cell suspension. Thereafter, CD45-positive cells were collected by magnetic separation using magnetic beads (MACS). Resulting 14 samples derived from different mice were stained, respectively, with the DNA-labeled antibodies in which 14 different kinds of single-stranded DNAs were used as labels. The cells were then washed with FACS buffer. The cells labeled with the antibody were counted using a flow cytometer, and then pooled at the same ratio. Using the BD Rhapsody System, the cells and beads were loaded on a microwell cartridge at appropriate densities according to the specification of Rhapsody. The cells were then lysed, and mRNA from each single cell and the label single-stranded DNA bound to the antibody bound to the cell were trapped on a single bead (comprising the Rhapsody universal adapter as a target-capturing nucleic acid) (target nucleic acid-capturing step). Reverse transcription reaction, and synthesis of the complementary strand of the antibody-labeling single-stranded DNA were carried out as recommended by the manufacturer (complementary-strand synthesis step), and then exonuclease I treatment was carried out to remove unreacted target-capturing nucleic acid on the beads (exonuclease treatment step).

After the exonuclease treatment, half of the beads (equivalent to about 9000 to 10,000 cells) were used to amplify total cDNA according to the amplification method of the present invention. The mRNA degradation step and the homopolymer addition step were allowed to proceed simultaneously by addition of RNase Hand TdT to the reaction liquid. In the homopolymer addition step, TdT reaction was carried out using ddCTP in an amount of ddCTP:dCTP = 1:20 to 1:40 in a reaction liquid comprising Tris-HCl, (NH₄)₂SO₄, KCl, K-phosphate, MgSO₄, CoCl₂, Triton X-100, and glycerol, to add poly(C) tails to the cDNA ends on the magnetic beads such that their lengths were limited.

After washing the beads, 5'BDWTA-dG9 primer and KAPA HiFi Hotstart Readymix, which is a high-fidelity PCR enzyme, were used to carry out the second-strand synthesis reaction. The number of times of annealing performed was 16.

After washing the beads, 1st PCR was carried out using NH2-5'BDWTA primer, NH2-universal primer, and Hashtag Common primer, and KAPA HiFi Hotstart Readymix. To 50 µL of the PCR reaction liquid, 32.5 µL of AmPure XP bead solution was added (bead concentration 0.65-fold), and the beads were collected by a magnet, followed by washing and elution, to obtain a cDNA fraction having a chain length of not less than 400 bp. Further, AmPure XP bead solution at 0.65-fold concentration was added to the supernatant (final concentration, 1.3-fold), and purification was carried out in the same manner to obtain an antibody-labeling DNA fraction having a chain length of 150 bp to 400 bp. After the purification, the cDNA fraction was subjected to 2nd PCR using NH2-5'BDWTA primer and NH2-universal primer, and KAPA HiFi Hotstart Readymix. The antibody-labeling DNA fraction was subjected to 2nd PCR using Hashtag common primer and NH2-universal primer, and KAPA HiFi Hotstart Readymix.

The DNA concentration and the size distribution in the 2nd PCR reaction liquid were investigated using an Agilent Bioanalyzer. As a result, a smear band centered at about 1 kbp was obtained for the cDNA fraction, and a single-peak DNA was obtained from the antibody-labeling DNA fraction having a uniform chain length (Fig. 13). The primer sequences used in the steps are as shown in Table 5. The obtained cDNA was fragmented using a NEBNext UltraII FS kit according to the protocol for the kit, followed by addition of an adapter, and addition of an illumina sequencing adapter and a barcode by PCR, to construct a library for sequence analysis. For the DNA-labeled antibody, addition of an illumina sequencing adapter and a barcode by PCR was carried out to construct a library for sequence analysis. The obtained libraries were quantified using a KAPA Library quantification kit for Illumina, and their size distributions were investigated using an Agilent Bioanalyzer. Sequencing was carried out using Illumina Novaseq 6000. The sequences of the adapter and the primers used for the preparation of the libraries for sequence analysis are as shown in Table 6.

**[Table 5]**

| Primer name | Sequence | SEQ ID NO |
|---|---|---|
| 5'BDWTA-dG9 | AAGCAGTGGTATCAACGCAGAGGGGGGGGG | 26 |
| NH2-5'BDWTA | /5AmMC6/AAGCAGTGGTATCAACGCAGA | 27 |
| NH2-Universal | /5AmMC6/ACACTCTTTCCCTACACGACGCTCTTCCGATCT | 14 |
| Hashtag Common | GTGACTGGAGTTCAGACGTGT | 28 |

| | | |
|---|---|---|
| 5AmMC6: 5' -(CH₂)₆-NH₂ modification | | |

**[Table 6]**

| Primer name | Sequence | SEQ ID NO |
|---|---|---|
| Illumina adapter | Phos-GATCGGAAGAGCACACGTCTGAACTCCAGTCAC-/AmM0/ T*CTAGCCTTCTCG | 15 |
| | | 16 |
| ILMN_UDI0030-I5 | AATGATACGGCGACCACCGAGATCTACACTGACAAGCCACTCTTTCCCTACACG*A*C | 29 |
| ILMN_UDI0033-I5 | AATGATACGGCGACCACCGAGATCTACACCCTGAACTACACTCTTTCCCTACACG*A*C | 30 |
| ILMN_UDI0030-I7 | CAAGCAGAAGACGGCATACGAGATTCCTGTAAGTGACTGGAGTTCAGACGT*G*T | 31 |
| ILMN_UDI0033-I7 | CAAGCAGAAGACGGCATACGAGATCCTCGGTAGTGACTGGAGTTCAGACGT*G*T | 32 |

| | | |
|---|---|---|
| * represents a phosphorothioate bond. Illumina adapter is an adapter prepared by annealing of two complementary primers. Phos: 5' phosphorylation. 3AmMO: 3' NH₂ modification The primers were used in the following combinations. ILMN_UDI0030-I5 and ILMN_UDI0030-I7 ILMN_UDI0033-I5 and ILMN_UDI0033-I7 | | |

The obtained single-cell transcriptome data were subjected to clustering based on the tag counts derived from the antibody-labeling DNA of each cell. As a result, it was found that the cells could be classified regarding from which sample they were derived, based on the expression level of the antibody-labeling DNA. The cell numbers of the different cells obtained were almost the same (Fig. 13). Cells from which two or more kinds of tags derived from the DNA-labeled antibody were sufficiently detected were identified as doublets (Fig. 13). Further, as a result of plotting of the numbers of genes detected from the cells of each sample, it was found that genes were almost uniformly detected among the 14 samples, and that the doublet samples showed higher numbers of detected genes compared to them (Fig. 13). Thus, the amplification method of the present invention used in combination with a DNA-labeled antibody was demonstrated to be useful in simultaneous single-cell transcriptome analysis for a plurality of samples.

## Claims

1. A method for nucleic acid amplification using a solid-phase carrier, the method comprising:
a target nucleic acid-capturing step of capturing a target nucleic acid comprising mRNA, on a solid-phase carrier through a target-capturing nucleic acid bound on the solid carrier;
a complementary-strand synthesis step of synthesizing a complementary DNA strand that is complementary to the target nucleic acid, said DNA strand comprising cDNA, from the target nucleic acid captured on the solid-phase carrier;
an exonuclease treatment step of degrading and removing, by exonuclease treatment, target-capturing nucleic acid on the solid-phase carrier that has not captured the target nucleic acid;
an mRNA degradation step of degrading the mRNA using a ribonuclease;
a homopolymer addition step of carrying out a reaction using terminal deoxynucleotidyl transferase in the presence of dCTP or dGTP, and a chain-terminating nucleotide triphosphate which is a chain-terminating CTP or a chain-terminating GTP, to add a nucleotide homopolymer which is poly(C) or poly(G) to an end of the complementary DNA strand;
a second-strand synthesis step of synthesizing a second strand for the complementary DNA strand bound on the solid phase, using a second-strand-synthesizing primer comprising a homopolymer portion complementary to the nucleotide homopolymer; and
a nucleic acid amplification step of carrying out nucleic acid amplification reaction using the DNA double strand synthesized on the solid-phase carrier as a template;
wherein the ratio of addition of a chain-terminating CTP and dCTP or a chain-terminating GTP and dGTP in the homopolymer addition step is 1:15 to 1:40, and
wherein the chain length of the complementary homopolymer portion of the second-strand-synthesizing primer is 6 to 13 bases.

2. The method according to claim 1, wherein the chain-terminating nucleotide triphosphate is:
(i) ddNTP, a ddNTP derivative, or 3'-dNTP (N is C or G), wherein optionally the ddNTP derivative is ddNTP in which the 3'-position is modified with an atomic group comprising no OH group; or
(ii) ddNTP (N is C or G).

3. The method according to claim 1 or 2, wherein the mRNA degradation step and the homopolymer addition step are carried out simultaneously.

4. The method according to any one of claims 1 to 3, wherein the target-capturing nucleic acid comprises nucleic acid comprising a poly(T) portion, wherein optionally the target-capturing nucleic acid further comprises a first adapter portion on the 5'-side of the poly(T) portion.

5. The method according to claim 4, wherein:
(i) the solid-phase carrier is a bead, and
the target-capturing nucleic acid comprises a bead identification barcode portion between the first adapter portion and the poly(T) portion; or
(ii) the solid-phase carrier is a plate, and
the target-capturing nucleic acid is immobilized on a plurality of compartments on the plate, and comprises a compartment identification barcode portion between the first adapter portion and the poly(T) portion.

6. The method according to any one of claims 1 to 5, wherein the second-strand-synthesizing primer comprises a primer comprising a second adapter portion on the 5'-side of the complementary homopolymer portion, wherein optionally the second-strand-synthesizing primer comprises a molecular barcode portion between the second adapter portion and the complementary homopolymer portion.

7. The method according to any one of claims 4 to 6, wherein, in the nucleic acid amplification step, nucleic acid amplification reaction is carried out using a primer targeting the first adapter portion and a primer targeting the second adapter portion, Wherein optionally:
the solid-phase carrier is a bead, and
the target nucleic acid-capturing step is carried out in a microwell or microdroplet.

8. The method according to claim 6, wherein, in the nucleic acid amplification step, nucleic acid amplification reaction is carried out using a primer targeting a T cell receptor constant region and a primer targeting the second adapter portion to amplify cDNA regions encoding a TCR variable region.

9. The method according to any one of claims 1 to 8,
wherein
the target nucleic acid comprises mRNA and an oligonucleic acid, said oligonucleic acid being bound to a specific-binding molecule;
the target-capturing nucleic acid comprises a nucleic acid comprising an mRNA-capturing sequence for capturing mRNA and a nucleic acid comprising an oligonucleic acid-capturing region for capturing the oligonucleic acid;
the oligonucleic acid comprises a capture target region whose sequence is complementary to the oligonucleic acid-capturing region; and
in the complementary-strand synthesis step, cDNA synthesis by reverse transcription reaction from the mRNA and complementary-strand synthesis from the oligonucleic acid are carried out, wherein optionally:
the capture target region is poly(A), and
the target-capturing nucleic acid comprises nucleic acid comprising a poly(T) portion as the nucleic acid comprising the mRNA-capturing region and as the nucleic acid comprising the oligonucleic acid-capturing region.

10. The method according to claim 9, wherein the specific-binding molecule is at least one kind of molecule that binds to a cell surface molecule directly, or indirectly through a specific-binding partner molecule.

11. The method according to claim 10, wherein
the specific-binding molecule is at least one kind of antibody or antibody fragment against the cell surface molecule.

12. The method according to claim 10,
wherein
the specific-binding molecule is an avidin-type molecule,
the specific-binding partner molecule is a biotin-type molecule, and
the method comprises, before the target nucleic acid-capturing step, the steps of:
treating a sample comprising a cell with a biotin-type molecule to generally label cell surface molecules, and then allowing the sample to react with an avidin-type molecule labeled with the oligonucleic acid, to indirectly bind the avidin-type molecule labeled with the oligonucleic acid to the cell surface; and
lysing the cell.

13. The method according to any one of claims 9 to 11, comprising, before the target nucleic acid-capturing step, the steps of:
bringing a sample comprising a cell into contact with the specific-binding molecule labeled with the oligonucleic acid, to directly or indirectly bind the specific-binding molecule to the cell surface; and
lysing the cell.

14. The method according to any one of claims 9 to 13,
wherein
the oligonucleic acid comprises a common sequence on the 5'-side of the capture target region, and
the target-capturing nucleic acid comprises the first adapter portion on the 5'-side of the poly(T) portion, wherein optionally the oligonucleic acid comprises a specific-binding-molecule-identification barcode portion between the common sequence and the capture target region.

15. The method according to claim 14, wherein the second-strand-synthesizing primer comprises:
a primer comprising the second adapter portion on the 5'-side of the complementary homopolymer portion; and
a primer comprising the common sequence, wherein optionally, in the nucleic acid amplification step:
(i) nucleic acid amplification reaction is carried out using a primer targeting the first adapter portion, a primer targeting the second adapter portion, and a primer comprising the common sequence, to simultaneously amplify cDNA and the oligonucleic acid, and then size fractionation of the amplification product is carried out to separate the amplified cDNA and oligonucleic acid from each other; or
(ii) nucleic acid amplification reaction is carried out using a primer targeting a T cell receptor constant region , a primer targeting the first adapter portion, a primer targeting the second adapter portion, and a primer comprising the common sequence, to amplify cDNA regions encoding a T cell receptor variable region and a complementary strand synthesized from the oligonucleic acid.

## Patentansprüche

1. Verfahren zur Nucleinsäure-Amplifikation unter Verwendung eines Festphasenträgers, wobei das Verfahren Folgendes umfasst:
einen Zielnucleinsäure-Einfangschritt des Einfangens einer Zielnucleinsäure, umfassend mRNA, auf einem Festphasenträger durch eine zieleinfangende Nucleinsäure, die auf dem festen Träger gebunden ist;
einen Komplementärstrang-Syntheseschritt des Synthetisierens eines komplementären DNA-Strangs, der zu der Zielnucleinsäure komplementär ist, wobei der DNA-Strang cDNA von der Zielnucleinsäure umfasst, die auf dem Festphasenträger eingefangen wurde;
einen Exonuclease-Behandlungsschritt des Abbauens und Entfernens der zieleinfangenden Nucleinsäure auf dem Festphasenträger, die die Zielnucleinsäure nicht eingefangen hat, durch Exonuclease-Behandlung;
einen mRNA-Abbauschritt des Abbauens der mRNA unter Verwendung einer Ribonuclease;
einen Homopolymer-Hinzufügungsschritt des Durchführens einer Reaktion unter Verwendung einer endständigen Desoxynucleotidyltransferase in Gegenwart von dCTP oder dGTP und eines kettenabschließenden Nucleotidtriphosphats, das ein kettenabschließendes CTP oder kettenabschließendes GTP ist, um ein Nucleotidhomopolymer, das Poly(C) oder Poly(G) ist, zu einem Ende des komplementären DNA-Strangs hinzuzufügen;
einen Syntheseschritt des zweiten Strangs des Synthetisierens eines zweiten Strangs für den komplementären DNA-Strang, der auf der festen Phase gebunden ist, unter Verwendung eines Synthetisierungsprimers für den zweiten Strang, umfassend einen Homopolymerabschnitt, der komplementär zu dem Nucleotidhomopolymer ist; und
einen Nucleinsäureamplifikationsschritt des Durchführens einer Nucleinsäureamplifikationsreaktion unter Verwendung des DNA-Doppelstrangs, der auf dem Festphasenträger synthetisiert wurde, als Matrize;
wobei das Verhältnis des Hinzufügens eines kettenabschließenden CTP und dCTP oder eines kettenabschließenden GTP und dGTP in dem Homopolymer-Hinzufügungsschritt 1:15 bis 1:40 beträgt und
wobei die Kettenlänge des komplementären Homopolymerabschnitts des Synthetisierungsprimers für den zweiten Strang 6 bis 13 Basen beträgt.

2. Verfahren nach Anspruch 1, wobei das kettenabschließende Nucleotidtriphosphat Folgendes ist:
(i) ddNTP, ein ddNTP-Derivat oder 3'-dNTP (N ist C oder G), wobei das ddNTP-Derivat gegebenenfalls ddNTP ist, in dem die 3`-Position mit einer Atomgruppe modifiziert ist, die keine OH-Gruppe umfasst; oder
(ii) ddNTP (N ist C oder G).

3. Verfahren nach Anspruch 1 oder 2, wobei der mRNA-Abbauschritt und der Homopolymer-Hinzufügungsschritt gleichzeitig durchgeführt werden.

4. Verfahren nach einem der Ansprüche 1 bis 3, wobei die zieleinfangende Nucleinsäure Nucleinsäure, umfassend einen Poly(T)-Abschnitt, umfasst, wobei die zieleinfangende Nucleinsäure gegebenenfalls weiters einen ersten Adapterabschnitt auf der 5'-Seite des Po-ly(T)-Abschnitts umfasst.

5. Verfahren nach Anspruch 4, wobei:
(i) der Festphasenträger ein Kügelchen ist und
die zieleinfangende Nucleinsäure einen Kügelchen-Identifikationsbarcodeabschnitt zwischen dem ersten Adapterabschnitt und dem Poly(T)-Abschnitt umfasst; oder
(ii) der Festphasenträger eine Platte ist und
die zieleinfangende Nucleinsäure auf einer Vielzahl von Abteilungen auf der Platte immobilisiert ist und einen Abteilungsidentifikationsbarcodeabschnitt zwischen dem ersten Adapterabschnitt und dem Poly(T)-Abschnitt umfasst.

6. Verfahren nach einem der Ansprüche 1 bis 5, wobei der den zweiten Strang synthetisierende Primer einen Primer, umfassend einen zweiten Adapterabschnitt, auf der 5`-Seite des komplementären Homopolymerabschnitts umfasst, wobei gegebenenfalls der den zweiten Strang synthetisierende Primer einen molekularen Barcodeabschnitt zwischen dem zweiten Adapterabschnitt und dem komplementären Homopolymerabschnitt umfasst.

7. Verfahren nach einem der Ansprüche 4 bis 6, wobei die Nucleinsäureamplifikationsreaktion in dem Nucleinsäureamplifikationsschritt unter Verwendung eines Primers, der auf den ersten Adapterabschnitt abzielt, und eines Primers, der auf den zweiten Adapterabschnitt abzielt, durchgeführt wird, wobei gegebenenfalls:
der Festphasenträger ein Kügelchen ist und
der Zielnucleinsäureeinfangschritt in einem Mikrowell oder einem Mikrotröpfchen durchgeführt wird.

8. Verfahren nach Anspruch 6, wobei die Nucleinsäureamplifikationsreaktion in dem Nucleinsäureamplifikationsschritt unter Verwendung eines Primers, der auf eine konstante Region eines T-Zell-Rezeptors abzielt, und eines Primers, der auf den zweiten Adapterabschnitt abzielt, durchgeführt wird, um cDNA-Regionen, die für eine variable TCR-Region kodieren, zu amplifizieren.

9. Verfahren nach einem der Ansprüche 1 bis 8, wobei:
die Zielnucleinsäure mRNA und eine Oligonucleinsäure umfasst, wobei die Oligonucleinsäure an ein spezifisch bindendes Molekül gebunden ist;
die zieleinfangende Nucleinsäure eine Nucleinsäure, umfassend eine mRNA-einfangende Sequenz zum Einfangen von mRNA, und eine Nucleinsäure, umfassend eine Oligonucleinsäure-einfangende Region zum Einfangen der Oligonucleinsäure, umfasst;
die Oligonucleinsäure eine Einfangzielregion umfasst, deren Sequenz komplementär zu der Oligonucleinsäure-einfangenden Region ist; und
in dem Komplementärstrangsyntheseschritt die cDNA-Synthese durch Umkehrtranskriptionsreaktion aus der mRNA und die Komplementärstrangsynthese aus der Oligonucleinsäure durchgeführt werden, wobei gegebenenfalls:
die Einfangzielregion Poly(A) ist und
die zieleinfangende Nucleinsäure eine Nucleinsäure, umfassend einen Poly(T)-Abschnitt, als die Nucleinsäure, umfassend die mRNA-einfangende Region, und als die Nucleinsäure, umfassend die Oligonucleinsäure-einfangende Region, umfasst.

10. Verfahren nach Anspruch 9, wobei das spezifisch bindende Molekül zumindest eine Art von Molekül ist, das direkt oder indirekt durch ein spezifisch bindendes Partnermolekül an ein Zelloberflächenmolekül bindet.

11. Verfahren nach Anspruch 10, wobei:
das spezifisch bindende Molekül zumindest eine Art von Antikörper oder Antikörperfragment gegen das Zelloberflächenmolekül ist.

12. Verfahren nach Anspruch 10, wobei:
das spezifisch bindende Molekül ein Molekül vom Avidintyp ist,
das spezifisch bindende Partnermolekül ein Molekül vom Biotintyp ist und
das Verfahren vor dem Zielnucleinsäure-Einfangschritt folgende Schritte umfasst:
Behandeln einer Probe, umfassend eine Zelle, mit einem Molekül vom Biotintyp, um Zelloberflächenmoleküle im Allgemeinen zu markieren, und dann Reagierenlassen der Probe mit einem Molekül vom Avidintyp, das mit der Oligonucleinsäure markiert ist, um das Molekül vom Avidintyp, das mit der Oligonucleinsäure markiert ist, indirekt an die Zelloberfläche zu binden; und
Lysieren der Zelle.

13. Verfahren nach einem der Ansprüche 9 bis 11, das vor dem Zielnucleinsäure-Einfangschritt folgende Schritte umfasst:
In-Kontakt-Bringen einer Probe, umfassend eine Zelle, mit dem spezifisch bindenden Molekül, das mit der Oligonucleinsäure markiert ist, um das spezifisch bindende Molekül direkt oder indirekt an die Zelloberfläche zu binden; und
Lysieren der Zelle.

14. Verfahren nach einem der Ansprüche 9 bis 13, wobei:
die Oligonucleinsäure eine gemeinsame Sequenz auf der 5`-Seite der Einfangzielregion umfasst und
die zieleinfangende Nucleinsäure den ersten Adapterabschnitt auf der 5'-Seite des Poly(T)-Abschnitts umfasst, wobei die Oligonucleinsäure gegebenenfalls einen Identifikationsbarcodeabschnitt für das spezifisch bindende Molekül zwischen der gemeinsamen Sequenz und der Einfangzielregion umfasst.

15. Verfahren nach Anspruch 14, wobei der den zweiten Strang synthetisierende Primer Folgendes umfasst:
einen Primer, umfassend den zweiten Adapterabschnitt, auf der 5'-Seite des komplementären Homopolymerabschnitts; und
einen Primer, umfassend die gemeinsame Sequenz, wobei in dem Nucleinsäureamplifikationsschritt gegebenenfalls:
(i) die Nucleinsäureamplifikationsreaktion unter Verwendung eines Primers, der auf den ersten Adapterabschnitt abzielt, eines Primers, der auf den zweiten Adapterabschnitt abzielt, und eines Primers, der die gemeinsame Sequenz umfasst, durchgeführt wird, um cDNA und die Oligonucleinsäure gleichzeitig zu amplifizieren, und dann eine Größenfraktionierung des Amplifikationsprodukts durchgeführt wird, um die amplifizierte cDNA und Oligonucleinsäure voneinander zu trennen; oder
(ii) die Nucleinsäureamplifikationsreaktion unter Verwendung eines Primers, der auf eine konstante Region eines T-Zellrezeptors abzielt, eines Primers, der auf den ersten Adapterabschnitt abzielt, eines Primers, der auf den zweiten Adapterabschnitt abzielt, und eines Primers, der die gemeinsame Sequenz umfasst, durchgeführt wird, um cDNA-Regionen, die für eine variable Region eines T-Zellrezeptors kodieren, und einen komplementären Strang, der aus der Oligonculeinsäure synthetisiert wurde, zu amplifizieren.

## Revendications

1. Procédé d'amplification d'acide nucléique à l'aide d'un support en phase solide, le procédé comprenant :
une étape de capture d'acide nucléique cible consistant à capturer un acide nucléique cible comprenant de l'ARNm, sur un support en phase solide par le biais d'un acide nucléique de capture de cible lié sur le support solide ;
une étape de synthèse de brin complémentaire consistant à synthétiser un brin d'ADN complémentaire qui est complémentaire à l'acide nucléique cible, ledit brin d'ADN comprenant de l'ADNc, à partir de l'acide nucléique cible capturé sur le support en phase solide ;
une étape de traitement avec une exonucléase consistant à dégrader et éliminer, par traitement avec une exonucléase, l'acide nucléique de capture de cible sur le support en phase solide qui n'a pas capturé l'acide nucléique cible ;
une étape de dégradation d'ARNm consistant à dégrader l'ARNm à l'aide d'une ribonucléase ;
une étape d'ajout d'homopolymère consistant à réaliser une réaction à l'aide d'une désoxynucléotidyl transférase terminale en présence de dCTP ou de dGTP, et d'un triphosphate nucléotidique de terminaison de chaîne qui est un CTP de terminaison de chaîne ou un GTP de terminaison de chaîne, pour ajouter un homopolymère nucléotidique qui est poly(C) ou poly(G) à une extrémité du brin d'ADN complémentaire ;
une étape de synthèse de second brin consistant à synthétiser un second brin pour le brin d'ADN complémentaire lié sur la phase solide, à l'aide d'une amorce de synthèse de second brin comprenant une partie homopolymère complémentaire à l'homopolymère nucléotidique ; et
une étape d'amplification d'acide nucléique consistant à réaliser une réaction d'amplification d'acide nucléique à l'aide du double brin d'ADN synthétisé sur le support en phase solide en tant que matrice ;
dans lequel le rapport d'ajout d'un CTP et d'un dCTP de terminaison de chaîne ou d'un GTP et d'un dGTP de terminaison de chaîne dans l'étape d'ajout d'homopolymère est de 1:15 à 1:40, et
dans lequel la longueur de chaîne de la partie homopolymère complémentaire de l'amorce de synthèse de second brin est de 6 à 13 bases.

2. Procédé selon la revendication 1, dans lequel le triphosphate nucléotidique de terminaison de chaîne est :
(i) ddNTP, un dérivé de ddNTP, ou 3'-dNTP (N est C ou G), dans lequel facultativement le dérivé de ddNTP est ddNTP dans lequel la position 3' est modifiée par un groupe atomique ne comprenant pas de groupe OH ; ou
(ii) ddNTP (N est C ou G).

3. Procédé selon la revendication 1 ou 2, dans lequel l'étape de dégradation d'ARNm et l'étape d'ajout d'homopolymère sont réalisées simultanément.

4. Procédé selon l'une quelconque des revendications 1 à 3, dans lequel l'acide nucléique de capture de cible comprend un acide nucléique comprenant une partie poly(T), dans lequel facultativement l'acide nucléique de capture de cible comprend en outre une première partie d'adaptation sur le côté 5' de la partie poly (T) .

5. Procédé selon la revendication 4, dans lequel :
(i) le support en phase solide est une bille, et l'acide nucléique de capture de cible comprend une partie de code à barres d'identification de bille entre la première partie d'adaptation et la partie poly(T) ; ou
(ii) le support en phase solide est une plaque, et
l'acide nucléique de capture de cible est immobilisé sur une pluralité de compartiments sur la plaque, et comprend une partie de code à barres d'identification de compartiment entre la première partie d'adaptation et la partie poly(T).

6. Procédé selon l'une quelconque des revendications 1 à 5, dans lequel l'amorce de synthèse de second brin comprend une amorce comprenant une seconde partie d'adaptation sur le côté 5' de la partie homopolymère complémentaire, dans lequel facultativement l'amorce de synthèse de second brin comprend une partie de code à barres moléculaire entre la seconde partie d'adaptation et la partie homopolymère complémentaire.

7. Procédé selon l'une quelconque des revendications 4 à 6, dans lequel, dans l'étape d'amplification d'acide nucléique, une réaction d'amplification d'acide nucléique est réalisée à l'aide d'une amorce ciblant la première partie d'adaptation et une amorce ciblant la seconde partie d'adaptation, dans lequel facultativement :
le support en phase solide est une bille, et
l'étape de capture d'acide nucléique cible est réalisée dans un micropuits ou une microgouttelette.

8. Procédé selon la revendication 6, dans lequel, dans l'étape d'amplification d'acide nucléique, une réaction d'amplification d'acide nucléique est réalisée à l'aide d'une amorce ciblant une région constante de récepteur de lymphocyte T et une amorce ciblant la seconde partie d'adaptation pour amplifier des régions d'ADNc codant pour une région variable de TCR.

9. Procédé selon l'une quelconque des revendications 1 à 8,
dans lequel
l'acide nucléique cible comprend de l'ARNm et un acide oligonucléique, ledit acide oligonucléique étant lié à une molécule à liaison spécifique ;
l'acide nucléique de capture de cible comprend un acide nucléique comprenant une séquence de capture d'ARNm pour capturer de l'ARNm et un acide nucléique comprenant une région de capture d'acide oligonucléique pour capturer l'acide oligonucléique ;
l'acide oligonucléique comprend une région cible de capture dont la séquence est complémentaire à la région de capture d'acide oligonucléique ; et
dans l'étape de synthèse de brin complémentaire, une synthèse d'ADNc par réaction de transcription inverse à partir de l'ARNm et une synthèse de brin complémentaire à partir de l'acide oligonucléique sont réalisées, dans lequel facultativement :
la région cible de capture est poly(A), et
l'acide nucléique de capture de cible comprend un acide nucléique comprenant une partie poly(T) en tant qu'acide nucléique comprenant la région de capture d'ARNm et en tant qu'acide nucléique comprenant la région de capture d'acide oligonucléique.

10. Procédé selon la revendication 9, dans lequel la molécule à liaison spécifique est au moins un type de molécule qui se lie à une molécule de surface cellulaire directement, ou indirectement par le biais d'une molécule partenaire à liaison spécifique.

11. Procédé selon la revendication 10, dans lequel
la molécule à liaison spécifique est au moins un type d'anticorps ou de fragment d'anticorps contre la molécule de surface cellulaire.

12. Procédé selon la revendication 10,
dans lequel
la molécule à liaison spécifique est une molécule de type avidine,
la molécule partenaire à liaison spécifique est une molécule de type biotine, et
le procédé comprend, avant l'étape de capture d'acide nucléique cible, les étapes de :
traitement d'un échantillon comprenant une cellule avec une molécule de type biotine pour marquer généralement des molécules de surface cellulaire, et ensuite fait de permettre à l'échantillon de réagir avec une molécule de type avidine marquée avec l'acide oligonucléique, pour lier indirectement la molécule de type avidine marquée avec l'acide oligonucléique à la surface cellulaire ; et
lyse de la cellule.

13. Procédé selon l'une quelconque des revendications 9 à 11, comprenant, avant l'étape de capture d'acide nucléique cible, les étapes de :
fait d'amener un échantillon comprenant une cellule en contact avec la molécule à liaison spécifique marquée avec l'acide oligonucléique, pour lier directement ou indirectement la molécule à liaison spécifique à la surface cellulaire ; et
lyse de la cellule.

14. Procédé selon l'une quelconque des revendications 9 à 13,
dans lequel
l'acide oligonucléique comprend une séquence commune sur le côté 5' de la région cible de capture, et
l'acide nucléique de capture de cible comprend la première partie d'adaptation sur le côté 5' de la partie poly(T), dans lequel facultativement l'acide oligonucléique comprend une partie de code à barres d'identification de molécule à liaison spécifique entre la séquence commune et la région cible de capture.

15. Procédé selon la revendication 14, dans lequel l'amorce de synthèse de second brin comprend :
une amorce comprenant la seconde partie d'adaptation sur le côté 5' de la partie homopolymère complémentaire ; et
une amorce comprenant la séquence commune, dans lequel facultativement, dans l'étape d'amplification d'acide nucléique :
(i) une réaction d'amplification d'acide nucléique est réalisée à l'aide d'une amorce ciblant la première partie d'adaptation, une amorce ciblant la seconde partie d'adaptation, et une amorce comprenant la séquence commune, pour amplifier simultanément l'ADNc et l'acide oligonucléique, et ensuite un fractionnement en fonction de la taille du produit d'amplification est réalisé pour séparer l'ADNc et l'acide oligonucléique amplifiés l'un de l'autre ; ou
(ii) une réaction d'amplification d'acide nucléique est réalisée à l'aide d'une amorce ciblant une région constante de récepteur de lymphocyte T, une amorce ciblant la première partie d'adaptation, une amorce ciblant la seconde partie d'adaptation, et une amorce comprenant la séquence commune, pour amplifier des régions d'ADNc codant pour une région variable de récepteur de lymphocyte T et un brin complémentaire synthétisé à partir de l'acide oligonucléique.
